# EUROPEAN PATENT APPLICATION

(11) **EP 2 033 578 A1**
(43) Date of publication of application: **11.03.2009**
(21) Application number: 07745091.4
(22) Date of filing: 12.06.2007
(51) Int. Cl.: A61B 5/151

(54) **LANCET ASSEMBLY**

(30) Priority: 13.06.2006 JP 2006163498
(71) Applicant: Izumi-Cosmo Company Limited, Osaka-shi, Osaka 530-0005 (JP)
(72) Inventor: KITAMURA, Yoritaka, Osaka-shi, Osaka 530-0005 (JP); ABE, Teruyuki, Chuo-ku, Tokyo 103-0022 (JP); SEKI, Kazuharu, Setagaya-ku, Tokyo 154-0001 (JP)
(74) Representative: Wiedemann, Peter
(86) International application number: PCT/JP2007/061804
(87) International publication number: WO 2007/145205

(57) **Abstract**

There is provided a pricking device which makes possible to isolate a distal end portion of a pricking element from its surrounding as much as possible while it is exposed from a lancet body, followed by taking out of an injector.

In a lancet assembly composed of a lancet 200 including a lancet body 204, a lancet cap 206 and a pricking member 210 and a lancet case 100 that houses a portion of the lancet,
the lancet body has a protruding portion 212 and a stopper 213 located behind the protruding portion;
the lancet case includes an abutting portion 122 and wings which extend backward and obliquely; and
when rear end portions 120 of the wings are fitted into sides of the case body 114, they are located along the sides of the case body.

## Description

### Technical Field

The present invention relates to a lancet assembly constituted from a lancet and a lancet case that houses the former, an injector used in combination with such lancet assembly, and a pricking device constituted from such lancet assembly and such injector. Such device is used in pricking a predetermined portion of a body with a sharp pricking member such as a needle for sampling a body fluid such as blood.

### Background Art

Various pricking devices have been used to take a small amount of blood for the purpose of measuring the blood sugar level of patients with diabetes. Such a device is constituted from a lancet having a pricking member (for example a needle formed from a metal) that pricks a predetermined portion of the body of a patient and an injector. The lancet is incorporated in the injector that launches the lancet with the pricking member exposed at the distal end thereof, the lancet being launched toward the predetermined portion by making use of the expanding action of a compressed spring provided in the injector.

When taking an amount of blood by using such a pricking device as described above, particular attention must be paid in the handling of the lancet that has been used. In the lancet that has been used, typically the distal end portion of the pricking member that bears a trace of the patient's blood is exposed from a lancet body. Should a portion of the body of a person other than the patient, for example a nurse who takes the blood sample, accidentally touches the distal end portion of the pricking member, the body portion may be pricked by the distal end portion of the pricking member causing a cut through which the patient's blood may enter the other person's body, thus posing the danger of infection of a disease.

Known pricking devices are not necessarily designed with due consideration given to the handling of the lancet that has been used. For example, it has been proposed to apply a cap on the exposed distal end portion of the pricking member after the pricking operation (refer to Patent Document 1 which will be mentioned later). This device requires the lancet to be carefully handle the lancet in the state of its distal end thereof exposed so as to apply the cap thereon, and therefore the danger described above is not sufficiently eliminated.

Accordingly, the pricking device requires utmost attention in handling the lancet after it has been used, and there is a demand for a pricking device that allows the lancet to be handled after safety thereof has been ensured.
Patent Document 1: U.S. Patent No. 5,385,571

### Disclosure of the Invention

### Problem to Be Solved by the Invention

It is an object of the present invention to provide a pricking device that allows a lancet to be removed from an injector after isolating a protruding distal end portion of a pricking member from its surrounding, rather than removing the lancet from the injector with the distal end portion of the pricking member remaining in the state of protruding from a lancet body, after pricking.

### Means to Solve the Problem

In the first embodiment, the present invention provides a lancet assembly comprising a lancet and a lancet case that houses a portion of the former, wherein
the lancet scomprises a lancet body, a lancet cap and a pricking member (generally, a pricking member made of a metal);
the lancet case comprises a case body having a front end opening and a rear end opening, and a pair of wings disposed on sides of the case body;
as for the lancet, the pricking member is disposed in the lancet body and the lancet cap while straddling over these members, and the distal end portion of the pricking member being enclosed by the lancet cap;
the lancet body has a protruding portion and a stopper located behind the protruding portion;
each wing comprises a front end portion and a rear end portion and an abutting portion between them, the front end portion is connected integrally to the case body and the rear end portion is able to be fitted into a side of the case body;
when the rear end portions of the wings are not fitted into the sides of the case body, the rear end portions are located outwardly from the case body, so that the wings protrude backward and obliquely from the sides of the case body; and
when the rear end portions of the wings are fitted into the sides of the case body, the wings extend along the sides of the case body (as a result, the rear sides of the abutting portions become able to contact the front sides of the protruding portions of the lancet body, and thereby the forward movement of the protruding portions is restricted). With such lancet assembly, it is that the lancet is a reson molded article (for example, an article produced by the injection molding of a resin).

In one embodiment of the lancet assembly of the present invention, the lancet cap and the lancet body are integrally connected to each other via a weakened portion, and a rear portion of the lancet cap that is located in front of the weakened portion, the weakened portion and a front portion of the lancet body that is located behind the weakened portion are housed in the lancet case as said portion.

In other embodiment of the lancet assembly of the present invention, the lancet cap and the lancet body may also be independent of each other, and in this case, the rear portion of the lancet cap and the front portion of the lancet body are disposed as said portions housed in the lancet case. In one embodiment, the lancet cap and the lancet body may be substantially adjacent to each other without being apart from each other. In another embodiment, these members may be apart from each other, and the pricking member may be partially exposed.

It is noted that when an outward foce is applied to the rear end portion of the wing which is fitted in the side of the case body, the rear end portion is elastically able to return to its original shape. That is, the wing substantially splays out.

In the second embodiment, the present invention provides an injector to which has the lancet assembly of the present invention described above or to be described later in detail is set therein, and launches the lancet body with the distal end portion of the pricking member being exposed, the injector comprising therein a plunger that launches the lancet body with the distal end portion of the pricking member being exposed and a member that expands the wing outward (namely a wing splaying member).

### Effects of the Invention

In the case wherein the lancet assembly of the present invention and the injector of the present invention are combined and used as the pricking device of the present invention, preparation for the pricking operation is completed only by merely inserting the lancet assembly so as to load it into the injector and twisting off and removing the lancet cap. The pricking device also allows it, after the pricking operation, to remove the distal end portion of the pricking member from the injector in a state of being substantially isolated in the lancet case from the surrounding, although it is still protruding from the lancet body. It is noted that "the state of being substantially isolated from the surrounding" means that the distal end portion of the pricking member is located in the lancet case at a position sufficiently away from the front end opening of the case body, and therefore a person who takes the blood sample does not normally touch the distal end portion in the routine pricking operations.

### Brief Description of Drawings

Fig. 1 is a schematic perspective view of a lancet assembly of the present invention.
Fig. 2 is a schematic perspective view of a lancet assembly of the present invention, with a near side half of the lancet case being cut away (wings are closed) for the ease of understanding the structure.
Fig. 3 is a schematic perspective view of a lancet that constitutes the lancet assembly of the present invention.
Fig. 4 is a schematic perspective view of a lancet case that constitutes the lancet assembly of the present invention.
Fig. 5 is a schematic perspective view of an injector into which the lancet assembly is loaded.
Fig. 6 is a schematic perspective view of the injector with the lancet assembly being loaded therein.
Fig. 7 is a schematic perspective view of the lancet case that constitutes the lancet assembly of the present invention viewed obliquely from the back (wings are open).
Fig. 8 is a schematic perspective view of the lancet case that constitutes the lancet assembly of the present invention viewed obliquely from the back (wings are closed).
Fig. 9 is a schematic perspective view of the lancet assembly of the present invention, with a near side half of the lancet case being cut away (wings are open) for the ease of understanding the structure.
Fig. 10 is a schematic perspective view of another embodiment of the lancet of the present invention.
Fig. 11 is a schematic perspective view of the state of the lancet assembly inserted and the lancet body put into contact with the front end portion of the plunger so as to load the lancet assembly into the injector, with a near side housing half of the injector housing being removed.
Fig. 12 is a schematic perspective view of the state of the lancet assembly inserted and the lancet body put into contact with the front end portion of the plunger so as to load the lancet assembly in the injector (the same state as that of Fig. 11), with near side halves of the lancet case, an ejector, a drum, etc. also being removed.
Fig. 13 is a schematic perspective view of the plunger.
Fig. 14 is a schematic perspective view showing the state of a rear end of the lancet body held by the plunger, similarly to Fig. 11.
Fig. 15 is a schematic perspective view showing the state of the rear end of the lancet body held by the plunger, similarly to Fig. 12.
Fig. 16 is a schematic perspective view showing the state of the plunger being fully inserted causing the wings of the lancet case to splay out, similarly to Fig. 11.
Fig. 17 is a schematic perspective view showing the state of the plunger being fully inserted causing the wings of the lancet case to expand, similarly to Fig. 12.
Fig. 18 is a schematic perspective view showing the state of loading of the lancet being completed (the launchable state), similarly to Fig. 11.
Fig. 19 is a schematic perspective view showing the state of loading of the lancet being completed (the launchable state), similarly to Fig. 12.
Fig. 20 is a schematic perspective view of the state of a housing half that constitutes the injector hosing.
Fig. 21 is a schematic perspective view showing the state of the lancet cap being twisted off, similarly to Fig. 12.
Fig. 22 is a schematic perspective view showing the state of a trigger button just having been pressed, similarly to Fig. 12.
Fig. 23 is a schematic perspective view showing the state of the pricking member just pricking a predetermined portion, similarly to Fig. 11.
Fig. 24 is a schematic perspective view showing the state of the pricking member just pricking a predetermined portion, similarly to Fig. 12.
Fig. 25 is a schematic perspective view showing the state of the pricking member having retracted after pricking a predetermined portion, similarly to Fig. 11.
Fig. 26 is a schematic perspective view showing the state of the pricking member having retracted after pricking a predetermined portion, similarly to Fig. 12.
Fig. 27 is a schematic perspective view showing the state of starting to discharge the lancet assembly after the pricking operation, similarly to Fig. 11.
Fig. 28 is a schematic perspective view showing the state of starting to discharge the lancet assembly after the pricking operation, similarly to Fig. 12.
Fig. 29 is a schematic perspective view showing the state of a stopper coming out in the course of discharging the lancet assembly, similarly to Fig. 11.
Fig. 30 is a schematic perspective view showing the state of the stopper getting out in the course of discharging the lancet assembly, similarly to Fig. 12.
Fig. 31 is a schematic perspective view showing the state of the lancet body departing from the plunger in the course of discharging the lancet assembly, similarly to Fig. 11.
Fig. 32 is a schematic perspective view showing the state of the lancet body departing from the plunger in the course of discharging the lancet assembly, similarly to Fig. 12.
Fig. 33 is a schematic perspective view showing the state of completing the discharge of the lancet assembly, similarly to Fig. 11.
Fig. 34 is a schematic perspective view showing the state of completing the discharge of the lancet assembly, similarly to Fig. 12.
Fig. 35 is a schematic perspective view of an ejector.
Fig. 36 is a schematic perspective view of the lancet assembly being discharged, with a near side of the lancet case being removed.
Fig. 37 is a schematic perspective view of a ring-shaped member and a hitting member.
Fig. 38 is a schematic perspective view of a rotation limiting member.

### [Description of Reference Numerals]

- 100, 100':: Lancet assembly
- 102:: Lancet case
- 104:: Front end opening
- 106:: Front end opening
- 107:: Rear end opening
- 108:: Rear end portion
- 110:: Front end portion
- 112:: Bump
- 114:: Case body
- 116:: Wing
- 117:: Rear end opening defining wall
- 118:: Front end portion
- 119:: Wall edge
- 120:: Front end portion
- 121:: Slit
- 122:: Abutting portion
- 124:: Sloped surface
- 126:: Rear portion surface
- 127:: Wing accommodating space
- 128:: Side surface
- 129:: Protruding portion
- 130:: Rear end surface
- 131:: Recess
- 132:: Rear end wall
- 133:: Thin wall portion
- 135:: Channel
- 140:: Rail
- 200:: Lancet
- 202:: Rear end portion
- 204:: Lancet body
- 206:: Lancet cap
- 207:: Top
- 208:: Weakened portion
- 209:: Protrusion
- 210:: Pricking member
- 212:: Protruding portion
- 213:: Stopper
- 215:: Protruding member
- 216:: Protruding portion
- 217,: 217': Fitting portion
- 218:: Bump
- 219:: Narrowing portion
- 221:: Rear surface
- 222:: Protruding portion
- 223:: Sloped surface
- 231:: Front side surface
- 300:: Injector
- 301:: Plate-like portion
- 302:: Front end opening
- 303:: Protrusion
- 304:: Protruding portion
- 306,: 308: Injector housing half
- 309:: Housing
- 310:: Plunger
- 312:: Ejector
- 314:: Front end portion
- 315:: Seat surface
- 316:: Recess
- 320, 322:: Leg
- 324:: Protruding portion
- 325:: Front side surface
- 326:: Rotary shaft
- 327:: Rear sloped surface
- 328:: Trigger lever 330: Shoulder
- 340:: Wedge-shaped member
- 341:: Front end portion
- 342:: Slope
- 344:: Front partition
- 345:: Opening
- 346:: Protrusion
- 347:: Rear side surface
- 348:: Recess
- 350:: Push button
- 351:: Base plate
- 352:: Pusher
- 354:: Operation button
- 355:: Channel
- 356:: Acting portion
- 357:: Return spring
- 358:: Rear partition
- 360:: Pricking depth adjusting drum
- 361:: Ring-shaped member
- 362:: Rear end portion of plunger
- 363:: Opening
- 365:: Hitting member
- 366:: Rotary knob
- 367, 367':: Hitting portion
- 371, 371', 373, 373':: step
- 380:: rotation limiting member
- 384:: First cylindrical portion
- 385:: Tapered portion
- 386:: Second cylindrical portion
- 388:: Chamfered portion
- 390:: Flange
- 391, 392, 393, 394, 395, 396, 397, 398:: Step

### Embodiments for Carrying Out the Invention

The lancet assembly of the present invention, the lancet and the lancet case that constitute the same, the injector that is used in combination with the lancet assembly, and a pricking device of the present invention will be described below in detail with reference to the accompanying drawings.

In this specification, directions and positions are described by using the words "front" and "rear". These words are used by making reference to a certain point (or a member or an element) which can be identified from the context, with the word "front" used to indicate a direction in which the pricking member moves to prick a predetermined portion from said certain point, namely the pricking direction, and the words "rear" is used to indicate the opposite direction. In this specification, directions and positions are described also by using the words "inward" and "outward". These words are used by making reference to a certain point (or a member or an element) which can be identified from the context, with the word "inward" used to indicate a direction toward the pricking member, and the words "outward" is used to indicate the opposite direction.

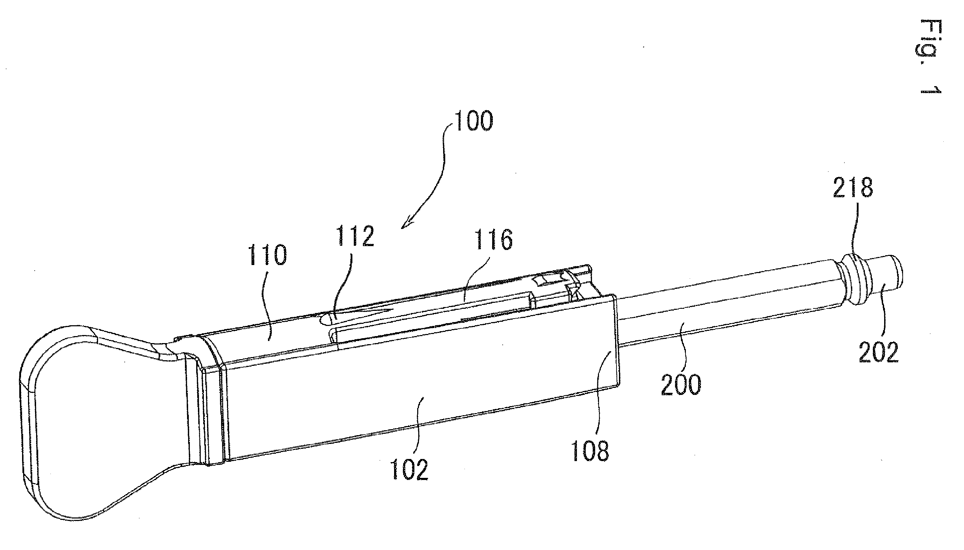

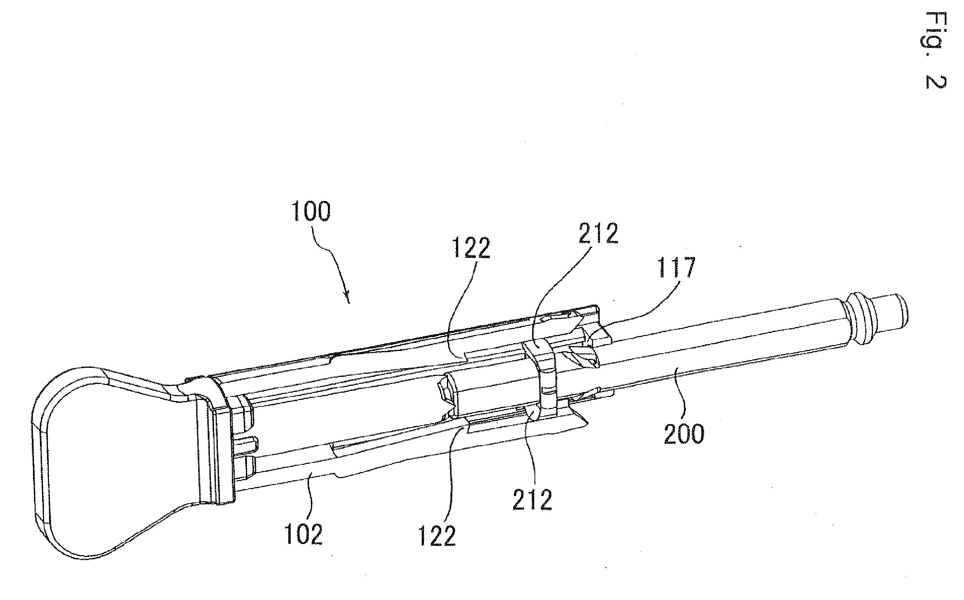

The lancet assembly 100 of the present invention is shown in a schematic perspective view of Fig. 1. The lancet assembly 100 is formed of the lancet case 102 and the lancet 200 that is partially housed in the former. For the readily understanding the structure of the lancet assembly, Fig. 2 shows the lancet case 102 with a near side half thereof cut away, in a schematic perspective view. These drawings show the lancet assembly in a complete state achieved by incorporating the lancet 200 in the lancet case 102. The lancet assembly 100 in the shown state can be inserted and set in the injector as described below, and is preferably put on the market in the illustrated state as shown in Fig. 1.

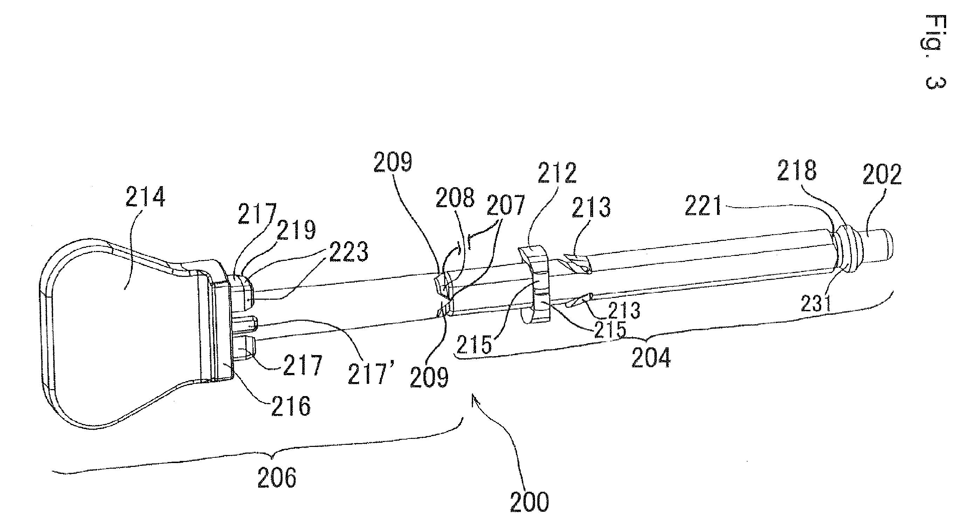

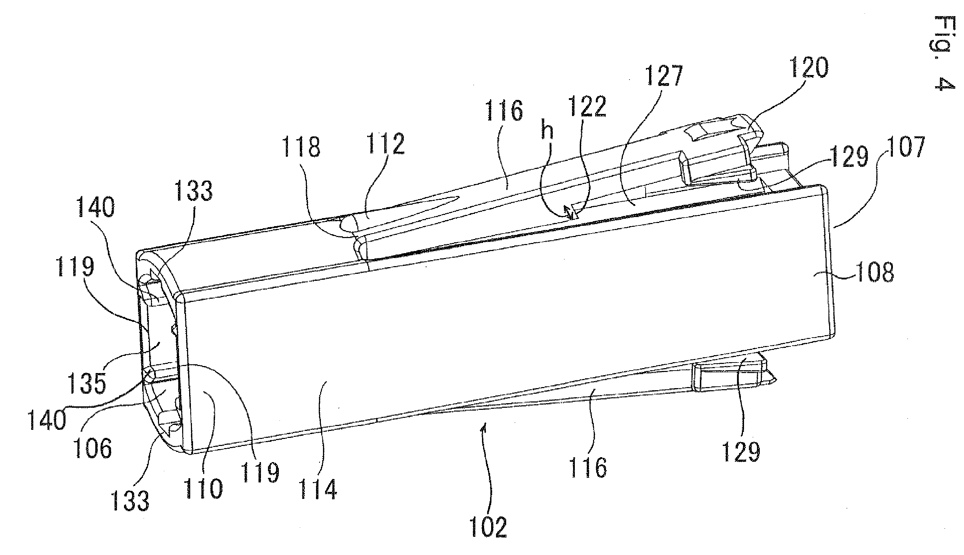

The lancet 200 and the lancet case 102 that constitute the lancet assembly shown in Fig. 1 and Fig. 2 are shown in Fig. 3 and Fag. 4 in schematic perspective view, respectively. Fig. 4 shows the lancet case different from that shown in Fig. 1 and Fig. 2 in that the wings 116 of the lancet case receives a force to elastically expand outward.

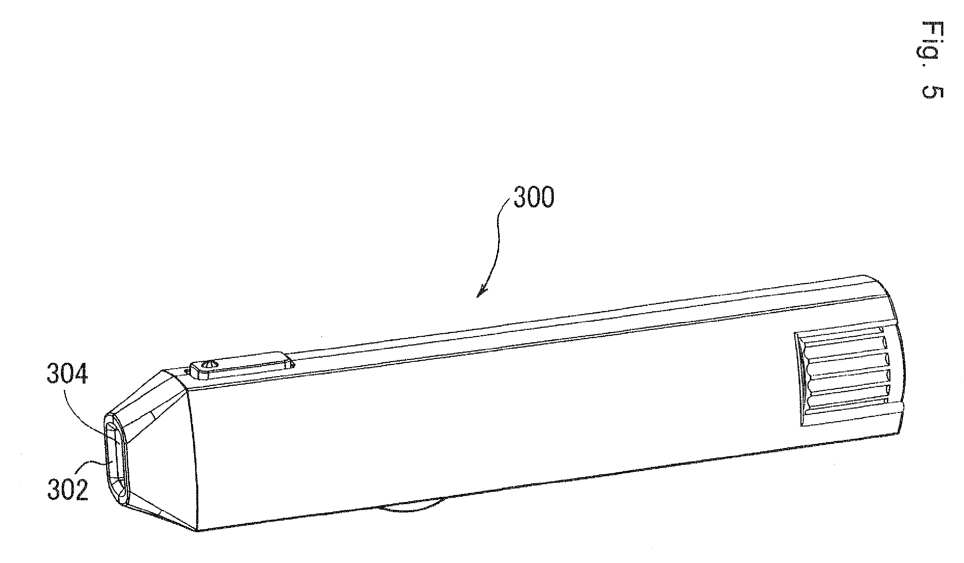

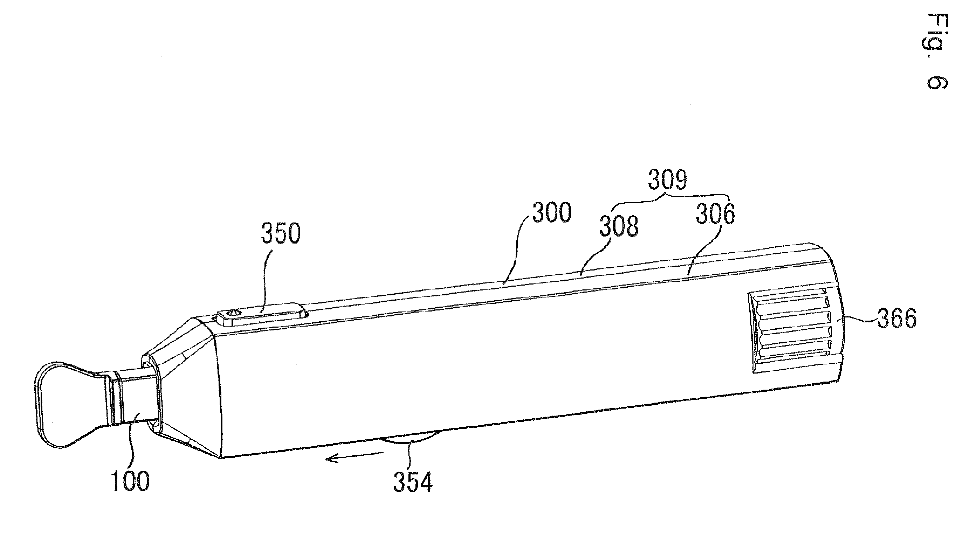

Fig. 5 shows, in a schematic perspective view, the injector 300 in which the lancet assembly is to be loaded, and Fig. 6 shows, in a schematic perspective view, the injector 300 in which the lancet assembly 100 has been set. The injector 300 houses most of a mechanism that loads the lancet assembly and prepares for launching the lancet body, a mechanism that launches the lancet body with a distal end portion of the pricking member exposed, and a mechanism that safely discharges the lancet assembly that has the lancet body with the distal end portion of the pricking member exposed, as will be described in detail later, in an inner space of a housing 309 delimited by injector housing halves 306 and 308. A trigger button 350, a discharge button 354 and a depth control knob 366 which will be described later are disposed outside of the housing 309.

The lancet assembly 100 is loaded in the injector 300 by first inserting the rear end portion 108 of the lancet case 102 through the front end opening 302 of the injector into the inside thereof (namely, toward the backward), and then inserting the most portion of the lancet case 102. The lancet case 102 has a bump (or protrusion) 112 provided on the outside of the rear portion of the front end 110. When the bump 112 has gotten over a protruding portion 304 provided on the wall that defines the front end opening 302 of the injector, or loading of the lancet assembly onto the injector is completed.

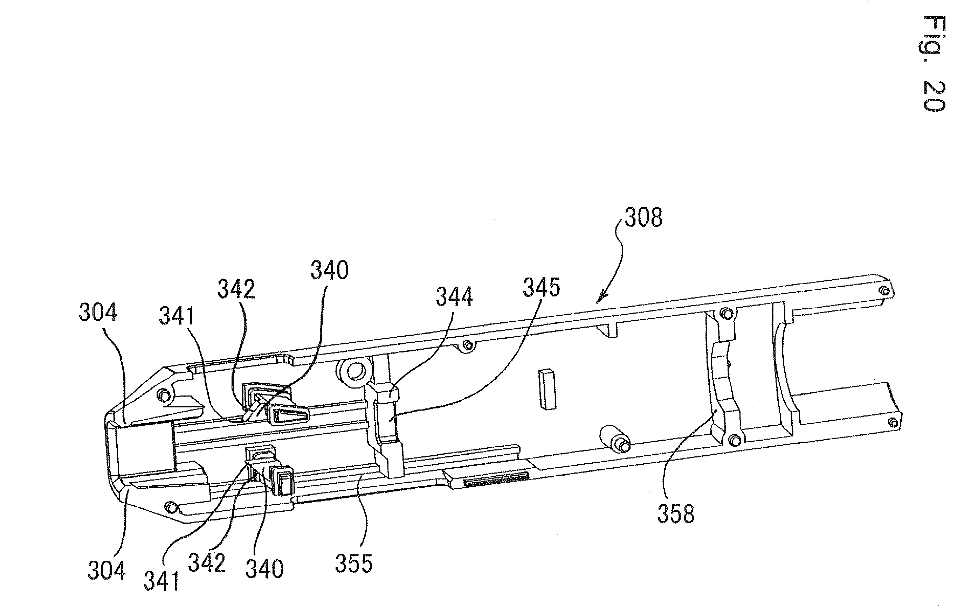

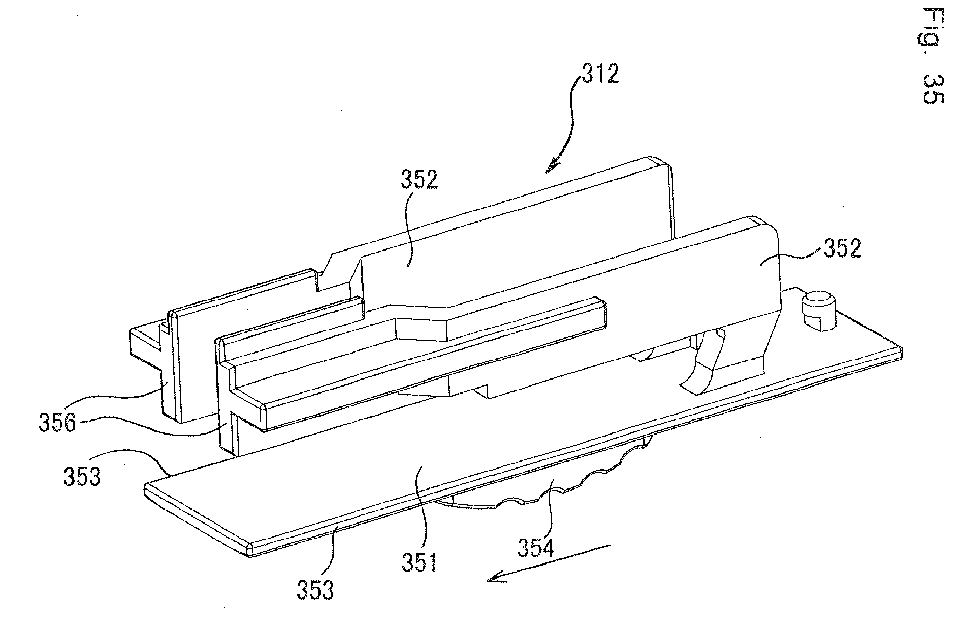

The state of completing of the loading as described above is shown in Fig. 6. In this state, the rear end portions 120 of the wings 116 of the lancet case 102 are in contact with the wing splaying member 340 (refer to Fig. 20) and thereby caused to expand (the state shown in Fig. 4). At this time, it is preferable that an acting portion 356 (refer to Fig. 35) of an ejector 312 is in contact with the rear end portion 108 of the lancet case.

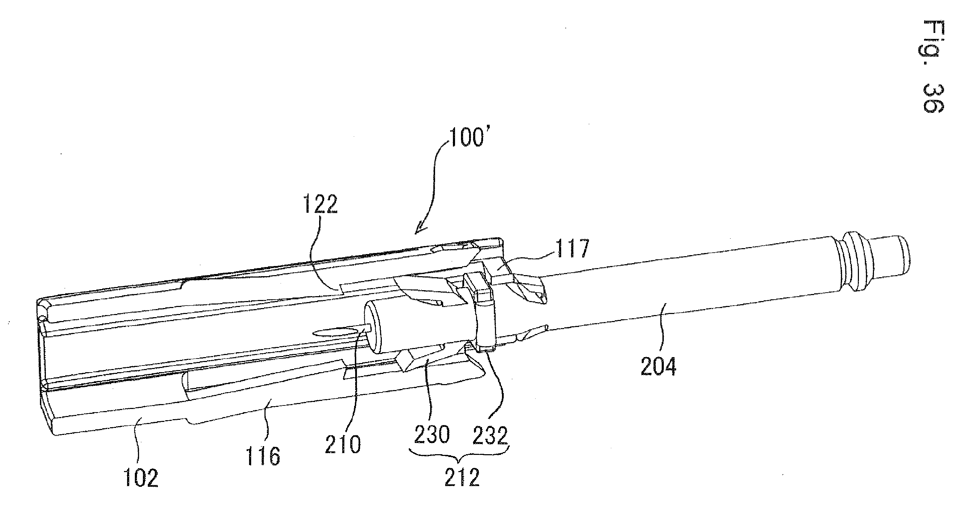

The lancet 200 that constitutes the lancet assembly of the present invention, shown in Fig. 3 in a perspective view, comprises the lancet body 204 and the lancet cap 206 which are integrally connected to each other via a weakened portion 208. In the lancet 200, the pricking member 210 (not shown in Fig. 3, with only a distal end portion thereof shown in Fig. 36) is disposed to extend in the lancet body 204 and the lancet cap 206 while straddling over these members, and the distal end portion of the pricking member 210 is enclosed by the lancet cap 206. Such lancet 200 is preferably a resin mold which is formed by molding a resin (a resin molded product) with the pricking member 210 inserted therein. The lancet body 204 has a protruding portions 212 on the outside thereof. The protruding portion 212 is preferably provided in a pair so as to oppose to each other with respect to the pricking member. The basic form of the lancet that comprises the lancet body 204 and the lancet cap 206 which are integrally connected to each other via the weakened portion 208 has already been known.

It is sufficient that the protruding portion 212 of the lancet body exists in a portion of the circumference of the lancet body, and it is preferable that the protruding portions are formed on portions of the circumference of the pricking member in a symmetrical relationship with respect the pricking member (for example, on the upper and lower sides of the pricking member as shown in Fig. 3). The protruding portion 212 is capable of restricting the forward movement of the lancet body in the lancet case by making contact of the wing 116 with the abutting portion 122 as will be described later, in the state of the wings 116 being fitted into upper and lower side surfaces of the case body 114 (the state shown in Fig. 2). The protruding portion 212 also abuts against the inside of the wall 117 that defines the rear end opening 107 of the lancet case so as to prohibit further backward movement thereof, when the lancet moves backward in the lancet case..

In another embodiment, the protruding portion 212 of the lancet body 204 may be composed of a plurality of separated protruding members provided along substantially the entire circumference of the lancet body as shown in Fig. 3. In this case, it is not necessary that all of the protruding members have the function of restricting the forward and backward movements as described above. For example, the movement may be restricted by protruding members provided on the upper and lower sides of the lancet body as described above, with the protruding members 215 located on the left side and the right side having other functions. Specifically, a protruding member that does not participate in the contact with the abutting portion of the wing, particularly the protruding member 215 that opposes to the inside of the side surface of the lancet body where no wing is provided, may be constituted so as to fit in a channel 135 (refer to Fig. 4) that is provided on the inside of the lancet body to extend along the pricking direction so as to be guided thereby, which would stabilize the forward and backward movements of the lancet body upon launching of the lancet.

As to such protruding portion 212 provided on the lancet body as described above, the protruding member is indispensable for the restricting the movement of the lancet body. However, the protruding member is preferably provided but may not necessarily be provided for guiding the movement of the lancet body to help stabilize.

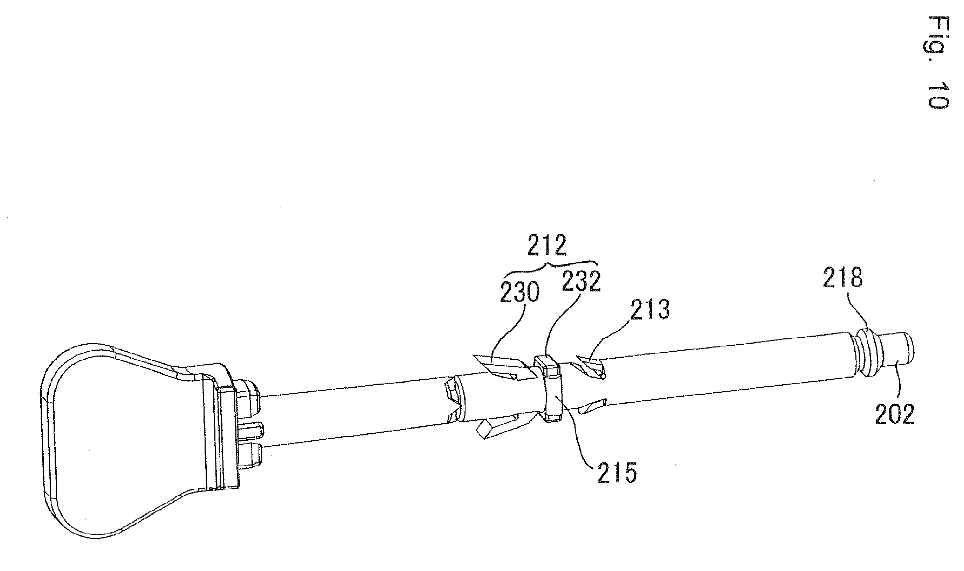

In the case the protruding portion comprises protruding members that have different functions, all of the protruding members may not necessarily be provided on the circumference at the same position of the lancet body with respect to the pricking direction. For example, the protruding member that restricts the movement of the lancet may be disposed at any appropriate position of the lancet body as long as it is ahead of the stopper 213, for instance ahead of the position shown in Fig. 3 (for example, at the position of the protrusion 230 provided in Fig. 10). In this case, a minimum length of the portion of the lancet body that portion protrudes from the rear end of the lancet case outward can be made larger, in the state of lancet assembly. When this is the case, the other protruding member 215 that has the function of guiding the movement of the lancet may be disposed at other position (for example, at the position of the protruding member 215 shown in Fig. 3 or at the position behind said position).

Hereinafter in this specification, the mere reference to "the protruding portion of the lancet body" is intended to mean the protruding member that has the function of preventing the movement.

Thus while the protruding portion 212 of the lancet body restricts the forward and backward movements of the lancet body within the lancet case, the protruding portion of the lancet body may be either constituted from protruding members provided at two different positions of the lancet body which are located apart from each other with respect to the pricking direction (namely apart from each other to the front and back). In this case, these protruding members have to be located ahead of the stopper 213.

Fig. 10 shows a lancet of such a form as described above, similarly to Fig. 3. The lancet shown in Fig. 10 is different from the lancet shown in Fig. 3 in that the protruding portion 212 is constituted from a pair of the upper and lower front protruding members 230 and a pair of the upper and the lower rear protrusing members 232 which pairs are separated at two positions with respect to the pricking direction. Shown in Fig. 10 is in a single member the protruding members 215 that have the guiding function (Fig. 3 two protruding members split into the upper and lower members). In the embodiment illustrated, the front side pair 230 abuts against the abutting portions 122 of the wings 116 so as to restrict the forward movement of the lancet body, while the rear side pair 232 abuts against the inside of the wall 117 that defines the rear end opening of the case body so as to restrict the backward movement of the lancet body.

There is not any specific restriction as to the form of the protruding portion 212 of the lancet body described above, as long as it can restrict the movement. Accordingly, there can be various forms (for example, a single form of pin, bar, ridge, etc.) or a combination of these forms. As will be described in detail later, the wings 116 are formed to be fitted in the side surface of the case body (or extend along the side surface). In thus fitted in state, there may happen such a case wherein the wing presses the protruding portion for some reason (for example, the wing which is to fit in abuts against the top of the abutting portion because of a somewhat larger height of the protruding portion due to dimensional variability caused in the manufacturing process). The protruding portion may also have such a form that decreases the height thereof (i.e. a distance to the top of the protruding portion from the surface of the lancet body) when the wing presses as described above (that is, a flexible form). For example, the protruding portion may have a form of a claw or fold-back similar to the stopper 213 described hereinafter. In this case, it is preferable that the distal end of the protruding portion, that is the claw or the like protrudes obliquely forward from the lancet body, as illustrated. In another embodiment, the protruding portion, especially the protruding member located on the front side, may have such a shape that extends from the lancet body in an L-letter configuration as illustrated, such as the protruding member 230 shown in Fig. 10. In this case, the distal end of the L shape is directed forward. The protruding member 232 located on the rear side may also have a shape similar to that of the front side, although it preferably has a form of bar or ridge, that is less flexible than the form of the claw, the L letter, or the fold-back, or a form of a claw with the distal end of the L-letter shape being directed rearward as illustrated.

The rear end portion 202 of the lancet body 204 is constituted so as to fit into the front end portion of the plunger 310 of the injector that launches the lancet, as will be described later, and the rear end portion 202 has a bump, preferably a bump 218 of a circumferential flange shape on the outside thereof, with the bump being adapted so as to fit in a recess 316 of a complementary shape provided on the inside of front end portion 314 (specifically on a leg to be described later) of the plunger 310. In place of the bump and the recess like thses, the bump may be used for the rear end portion of the lancet body and the recess may be used for the front end portion of the plunger.

The lancet body 204 further comprises a surface 221 that opposes to the front side surface of the bump 218. This rearward surface 221 comes into close contact with a seat surface 315 provided on the distal end of the plunger when the rear end portion 202 of the lancet body is grasped by the plunger 310.

The lancet body 204 has a pair of stoppers 213 provided behind the rear of the protruding portion 212. The stoppers have a function of preventing the entry of the stoppers through the rear end opening 107 of the case body 114 after getting out of the rear end opening 107 of the case body 114. That is, with respect to the movement of the lancet body 204 through the rear end opening 107 of the case body 114, the movement in only one direction (namely rearward movement) is allowed and the stoppers 213 can get out of the lancet case through the rear end opening 107 to the outside. However, it is impossible to get from the outside into the inside of the lancet case. As a result, movement of the lancet body in the other direction (namely forward movement) is restricted.

Such stoppers 213 preferably has a form of a protruding portion that extends from the lancet body 204 forward in an outwardly oblique direction, preferably the form of a claw or a fold-back, and are preferably provided in a pair on both sides of and through the pricking member. In another embodiment, the stopper may have an L-letter shape similarly to the protruding member 230 as described above. The stoppers of such a form are designed so as to function as follows: the portion of the lancet body that portion has the stoppers can move from the inside of the lancet case through the rear end opening 107 rearward to the outside, but once the stoppers have passed through the rear end opening, cannot get from the outside of the lancet case through the rear end opening forward into the inside of the lancet case because the distal end portions of the stoppers 213 abut against the outside of the wall that defines the rear end opening 107 and, when the lancet body is subjected to a force acting forward intended to move forward in this state, the stoppers expands further outward. As a result, the stoppers act to prevent the movement only in one direction. By forming the lancet from a resin, the stoppers are caused to function further effectively by making use of the elasticity of the former.

For example, by moving the lancet case forward relative to the lancet body when discharging the lancet assembly from the injector after its use, the lancet body is restricted from moving forward as the stoppers of the lancet body cannot enter the lancet case after the stoppers have been moved through rear end opening to the outside, as will be described later. Since the lancet body is not capable of moving forward, the lancet assembly cannot be reused for the pricking operation once it has been used.

In one preferred embodiment, the lancet cap 206 has a protruding portion 216 that protrudes outward, preferably a protruding portion 216 that extends over substantially the entire circumference at a predetermined position with respect to the pricking direction of the lancet cap. In other embodiment, protruding portions may be provided at two positions that oppose to each other via the pricking member (for example, the upper and lower sides of the lancet cap) or more positions. The protruding portion has an external profile larger than the inner profile of the front end opening of the case body, so that the protruding portion 216 cannot enter the inner space of the lancet case 102 through the front end opening 106. As a result, when the lancet is inserted into the lancet case to form the lancet assembly, the protruding portion abuts against the front end portion of the lancet case so as to prevent the lancet from moving rearward further with respect to the lancet case.

The front portion 214 of the lancet cap 206 has a grip that can be held to break the weakened portion 208 (if any), and the protruding portion 216 is provided behind thereof. The protruding portion 216 preferably extends in the form of a flange provided around the lancet cap, or a bar or ridge provided on the side as illustrated. The protruding portion is adapted to abut against the wall that defines the front end opening 106 of the case body so that the lancet 200 is unable to move further backward relative to the lancet case 102. The grip 214 can be held by fingers to force it through the front end opening of the injector and retract for setting the lancet assembly into the injector 300. Accordingly, the front portion 214 may be formed in a flat configuration as illustrated.

It is preferable that the lancet cap has a fitting portion 217 provided behind the protruding portion 216 described above. The fitting portion is a portion located around the lancet cap, and an outermost portion of the profile of the fitting portion (cross section perpendicular to the pricking direction thereof) has such a shape that fits into the front end opening 106 of the case body. As a result, when the lancet cap is inserted into the lancet case, the fitting portion fits into the lancet case and the protruding portion 216 of the lancet cap resides in front of the front end opening 106 of the case body while being adjacent thereto.

In the case where there is such a fitting portion, it is advantageous in that, when the lancet cap is inserted into the lancet case to assemble the lancet assembly, temporary fixation can be achieved without unnecessary movement of the lancet cap in the lancet case as the fitting portion is fitted in the lancet case. There is no restriction on the form and the number of the fitting portion as long as the fitting portion can fitted into the front end opening of the case body so as to temporarily fix the lancet.

The fitting portion described above preferably has such a form that narrows rearward. With this configuration, the fitting portion located behind the protruding portion has at least one surface that inclines, preferably so as to approach the pricking member, toward the back (namely a sloped surface). When the sloped surface is rotated around the pricking member, the surface moves so as to rise over an edge of the wall that defines the front end opening of the case body. As a result, the lancet cap moves forward relative to the lancet case, when the lancet cap is turned (twisted) relative to the lancet case around the extending direction of the pricking member. This movement causes the weakened portion to receive forces effecting both rotation and pull off, and therefore assists breaking of the weakened portion 208. With this regard, the sloped surface is preferably formed so as to make contact with the edge of the wall that defines the front end opening of the case body when the lancet cap is turned around the pricking direction.

As shown in Fig. 3, the fitting portion 217 has a narrowing portion 219 directed rearward, so that the fitting portion located behind the protruding portion has at least one sloped surface 223 that inclines so as to approach the pricking member toward the back. When the lancet cap 206 is turned relative to the lancet body 204, and therefore relative to the lancet case 102 so that the sloped surface turns around the pricking member in order to break the weakened portion, the sloped surface 223 rises over the edge 119 of the wall that defines the front end opening of the case body. As a result, twisting action causes the lancet cap to move relative to the lancet case.

The fitting portion located behind the protruding portion 216 may be either an extension of the rear portion of the protruding portion (hence integral with the protruding portion) as illustrated, or an independent portion spaced apart from the protruding portion. The narrowing form, when it is provided, may be such a form as a portion or whole of the fitting portion that narrows as described above. The lancet of the present invention shown in Fig. 3 has two fitting portions 217 (provided on the upper and lower sides of the protruding portion of the lancet cap in the rear portion thereof in the illustrated embodiment) that have the narrowing portions 219 in contact with the rear side of the protrusion 216, with a fitting portion 217' of the ordinary protrusion shape located therebetween. It is preferable that the narrowing portion of the fitting portion is formed so that the surface thereof that would oppose to the inner side surface of the lancet case forms the sloped surface 223.

In one embodiment of the present invention, it is preferable that the fitting portion of the lancet cap has such a form that is press-fitted in the front end opening of the case body, and the protruding portion of the lancet cap is held in the condition of abutting against the front end opening of the case body in the state of lancet assembly. In this case, when the fitting portion has the narrowing form in a portion thereof, the sloped surface that defines it assists press-fitting of the fitting portion in the front end opening of the case body.

When the fitting portion that is fitted as described above is provided, the lancet is temporarily fixed within the lancet case in the state shown in Fig. 1 or Fig. 2, when the lancet assembly is assembled. This is advantageous because, in this state, it is difficult to twist off the lancet cap (although it is not absolutely impossible to twist it off), so that misuse or accidental pricking do not occur thus achieving higher safety.

In the lancet 200 as described above, the lancet body 204 and the lancet cap 206 are connected such that the weakened portion 208 is broken by applying such a force that the lancet body 204 and the lancet cap 206 turn in the opposite directions around the extending direction of the pricking member 210, or applying such a force that the lancet body and the lancet cap move away from each other along an extending direction of the pricking member. Then, when the lancet cap 206 is moved away from the lancet body 204, the distal end of the pricking member 210 is exposed from the lancet body.

In the embodiment illustrated, the case body 114 of the lancet case 102 has a form of square cylinder as a whole. The lancet assembly 100 can be assembled by inserting the rear end 202 of the lancet 200 backward through the front end opening 106 of the case body to the inside thereof. The lancet case 102 has openings 106 and 107 at both ends of the case body 114 that constitutes the lancet case. During the pricking operation, a predetermined portion of human body (for example, a finger tip) to be pricked is applied onto the front end opening 106.

The lancet case 102 that constitutes the lancet assembly of the present invention comprises the case body 114 and the wing 116 disposed on the side surface thereof. The wings 116, which may be called arm-like member, is elongated members that extends obliquely and backward from the side surface of the case body, and it is preferably provided in a pair on the opposing side surfaces of the lancet case. The wings 116 have abutting portions 122 that protrude inwardly between the front end portion 118 and the rear end portion 120 thereof. The front end portion 118 is integrally connected to the case body 114, and the rear end portion 120 is detached from the case body in a free state, that is, the state wherein the wing 116 splays out. It is noted that wing 116 is in the state wherein the wing 116 splays out a little outward from the case body as shown in Fig. 4 unless a force is applied from the outside, the wings 116 of the case body 102 assume a state of splaying outwardly as shown in Fig. 4.

The lancet case is preferably a molded article of a resin. When a force is applied from the outside on the wings 166 to get in the state wherein the rear ends of the wings are fitted in the side of the case body as shown in Fig. 1, the wings 116 are in the state wherein they have been elastically deformed so as to extend alont the sides of the case body from the state wherein they are splayed out. The case body has spaces 127 formed in the sides thereof for accommodating the wings. The space is so sized that substantially the entire wing 116 can be accommodated, and the outer side surface of the wing 116 forms a substantially single side surface of the lancet case together with the lancet body (that is, the wing forms a portion of such side surface). That is, it is preferable that the wings do not protrude from the side surface of the lancet case as shown in Fig. 1. The case body, in particular, preferably has a space that accommodates the wing in just fit, which makes it possible for the fitted in wing and the case body together to define the side surface of the lancet case. As will be apparent from the drawing, this space constitutes a portion of the inner space of the lancet case.

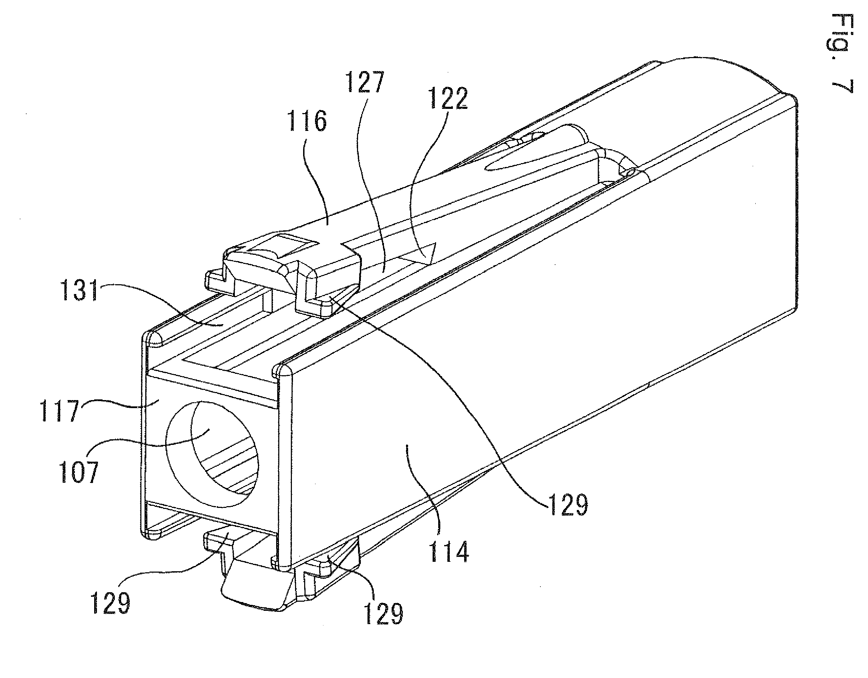

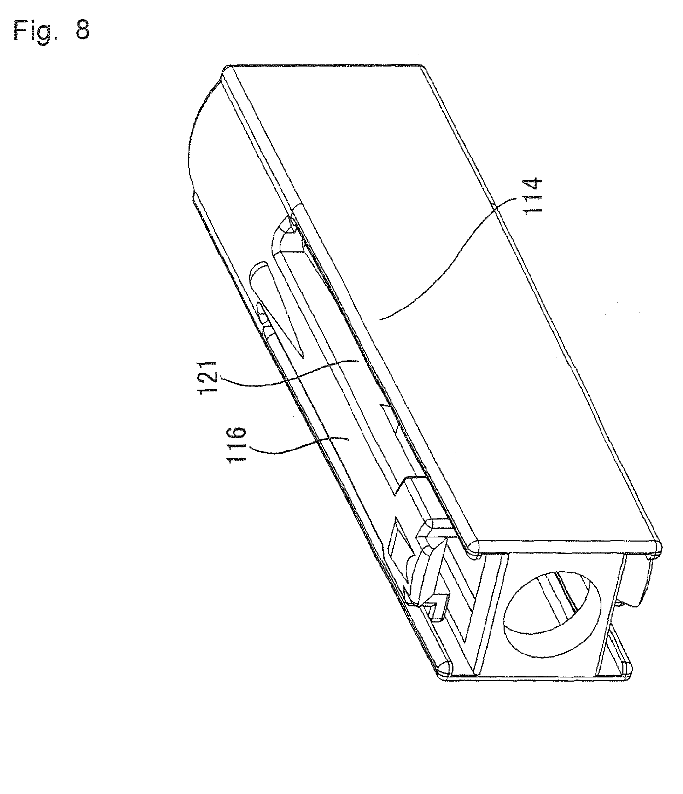

When the wing is located along the side of the case body as shown in Fig. 1, it is preferable that the wing is in the state wherein the wing is fitted into the side of the case body due to application of a force to the wing, for example to its rear end portion which force is applied against a force of the wing to attempt to flare outward (an elastic force derived from a resin). Such force that is applied to the rear end portion of the wing is for example a friction force generated between the rear end portion of the wing and the case body (for example, by the rear end portion being press fitted into the side of the case body), or such force is generated for example by the rear end portion of the wing being fitted into a recess that the side of the case body includes (for example, the rear end portion of the wing is snap fitted into such recess of the case body). Thus, when such force is releases in the state wherein such force is being applied, the wing returns to its original state which is in the splayed out form, that is, the form of the wing elastically changes to its original form. Figs. 7 and 8 show the state wherein the wings 116 are located away from the case body114 (i.e. the state wherein the wings 116 have splayed out) and the state wherein the wings 116 are fitted into the sides of the case body 114 (i.e. the state wherein the wings 116 have been closed) respectively when viewing the case body from its rearside obliquely.

Fitted of the wing 116 into the receiving space 127 is preferably achieved by the snap fit or the press fit of the whole of the part (for example, the rear end portion) of the wing. In the latter case, the wing 116 has a protruding portion 129 in its side, and the receiving space 127 includes a recess 131 as a space into which such protruding portion is fitted. Also, the protruding portion 129 preferably has a shapes which gradually flares rearward, specifically the triangle wing as shown. In this case, the fitting of the rear end portion of the wing into the side of the case body gets easy, and also an outward force which is required to be applied to the rear end portion so as to allow the wing to return its original shape from its being fitted into the side.

It is noted that in the lancet case as shown, the width of the rear end portion of the wing is larger, and the width of the wing ahead of the rear end portion is smaller a little. As a result, there is formed small slits 121 between the case body and the both sides of the wing (see Fig. 8). In this embodiment, the outer surface of the wing 116 and the side surface (the side surface which include the wing) of the case body 114 defines the same single surface (a little curved surface).

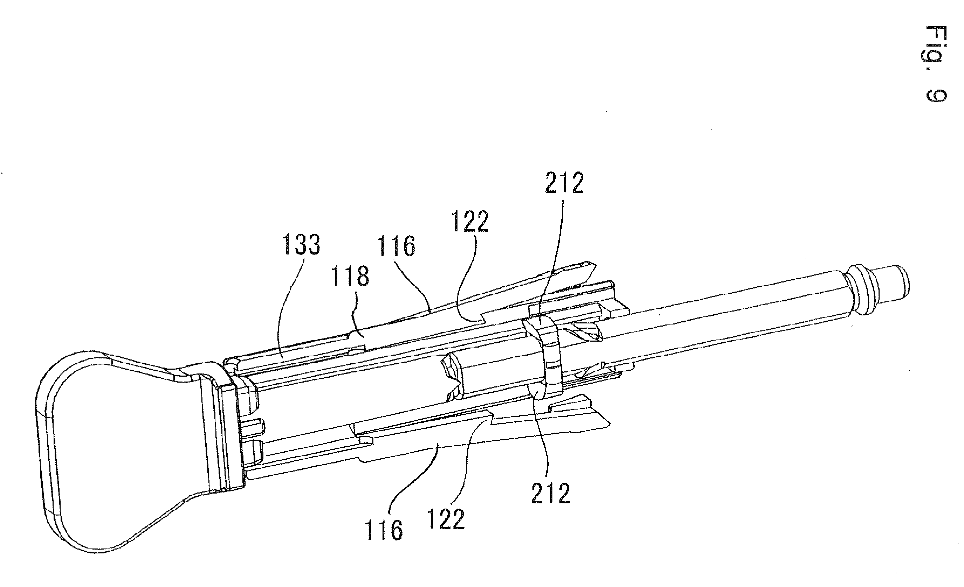

The state of the lancet assembly according to the present invention wherein the wings 116 are splayed out is shown in Fig. 9 similarly to Fig. 2. As seen from the drawing, in the shown state, the protruding portion 212 of the lancet body can move forward without hitting the abutting portion 122 of the wing 1116.

In the state wherein the wings 116 are fitted into the receiving space 127, the wings are elastically deformed as described above, and strictly the wings may be curved a little. In order that such curvature is minimized as much as possible, a wall portion located ahead of the front end portion of the wing which wall portion defines the case body is preferably thinner than the other portion of the wall, and in addition to this, it is more preferable that the wing is thinner. The provition of the thinner wall portion in the case body prevents the wing from being substantially curved so that substantially the whole of the wing is located along the side of the case body when the rear end portion of the wing is fitted into the side of the case body. For example, the wall portion which forms the portion ahead of the front end portion 118 of the wing 116 is formed to have a thinner wall thickness portion 133 as shown in Fig. 9. In the shown embodiment, the thinner wall thickness portion is formed from the front end portion 118 of the wing 118 to the front end opening 106.

In place of and/or in addition to the application of the force to the rear end portion of the wing as describd above, in order that the wing is fitted into the side of the case body, the wing may be designed such that a force is applied to a portion of the wing , for example a portion located ahead of the rear end portion rather than the rear end portion. In a further embodiment, such force is applied to substantially the entire wing. Thus, in other words, such force is applied to at least a portion of the wing.

It is noted that the lancet case preferably have a channel 135 on a side surface inside the case body 114 (a side surface without a wing), the protruding member 215 is guided along and in the channel. As described above, the lancet body 204 may comprise the two kinds of the protruding members 212 and 215, and these protruding members may be located at the same position or different positions with respect to the direction in which the pricking member extends. The embodiment shown in Fig. 3 corresponds to the former, and the embodiment shown in Fig. 10 corresponds to the latter in which the protruding members 230 and the protruding members 215 are in the forth and back relationship, and further the protruding member 212 which restricts the movement is in the forth and back relationship of the protruding members 230 and the protruding members 232.

The lancet case described above is preferably constituted by integrally forming the case body and the wings by molding (particularly injection molding) from a resin such as a polypropylene resin, a polyethylene resin, a polystyrene resin, a POM resin (polyacetal resin), a nylon resin, an ABS resin, a polycarbonate resin, a vinyl chloride resin, an elastomer resin, a silicone resin, a rubber based resin, a PBT resin (polybutylene terephthalate resin), a polyester copolymer resin or the like. As for the lancet, the pricking member is usually made of a metal, for example, a stainless steel, and the other portion may be formed of a resin similar to that of the lancet case, and is preferably formed by the injection molding with the pricking member being inserted.

The lancet assembly composed of the lancet and the lancet case as described above is set into the injector, and a predetermined portion is pricked. In the lancet assembly 100 that is assembled as shown in Fig. 1 or Fig. 2 (i.e. the wings are closed), the lancet 200 does not get out of the front end opening 106 of the case body 114 even when the lancet 200 receives a force directed forward by the actions of the protruding portion 212 of the lancet body and the abutting portions 122 of the wings 116, and also does not get out of the rear end opening 107 even when the lancet 200 receives a force directed rearward by the actions of the wall 117 that defines the rear end opening 107 and the protruding portions 212.

In the embodiment of the lancet shown in Fig. 10, the function of preventing the lancet from getting out of the front end opening 106 is performed by the protruding portions 230 and the function of preventing the lancet body from getting out of the rear end opening 107 is performed by the protruding portions 232. These protruding portions 230 and 232 form the protrusions 212. Therefore, when the wings 116 are closed, the protruding portions restrict the forward and backward movements of the lancet body 204 within the lancet case 102 similarly to the case of the lancet shown in Fig. 3 also in the lancet shown in Fig. 10.

With the lancet assembly of the present invention as described above, in the state wherein the lancet is inserted into the lancet case, the weakened portion is positioned in the lancet case, and the protruding portion of the lancet body is disposed between the abutting portions of the wings of the lancet case and the rear end opening of the case body,
(a) a portion (rear portion) of the lancet cap and a portion (front portion) of the lancet body are accommodated in the lancet cap (that is, a portion of the lancet is accommodated);
(b) in the state wherein the wings of the lancet case are fitted in the sides of the case body, the protruding portions of the lancet body can move back and forth between the abutting portions of the wings and the rear end opening of the case body but is prevented from moving further forward by abutting against the abutting portion, and is prevented from moving further rearward by the abutment against the wall that defines the rear end opening of the case body (refer to Fig. 4); and
(c) in the state wherein the wings of the lancet case have expanded outward from the sides of the case body, the protruding portions of the lancet body do not contact the abutting portions of the wings so as to become capable of moving forward from the abutting portions (refer to Fig. 9).

When the lancet assembly 100 that has been assembled as shown in Fig. 1 or Fig. 2 is inserted through the front end opening 302 of the injector 300 shown in Fig. 5, and the protruding portion 112 of the lancet case gets over the protruding portion 304 provided immediately behind the front end opening of the injector, the state shown in Fig. 6 is achieved, so that the lancet case and the injector are engaged or fitted with each other and the set-up of the lancet assembly in the injector completes. Instead of the protruding portion 304, a recess in which the protruding portion 112 is fitted may also be provided inside of the front end opening of the injector. That is, the set-up is ensured by protruding such two elements which engage or fit with each other to the lancet case and the injector (for example, the protrusion of the lancet case and the recess of the injector).

When the lancet assembly in such a state that the rear end portions of the wings are fitted into the sides of the case body and the entire wings extend along the sides of the case body is inserted through the front end opening into the injector, forces acting on the rear end portions of the wings are released by members that splay the wings outwardly (namely, wing splaying members, for example wedge-shaped members) provided inside of the injector, so that the wings deform to the state of being expanded outwardly. Such splaying members may apply the forces onto not only the rear end portion of the wing, but also the portion located in front thereof.

The lancet is grasped by the plunger of the injector when the lancet assembly of the present invention is loaded on the injector that is combined therewith. Then the relationship between the plunger and the trigger lever changes to "the state of the trigger lever being capable of holding x the plunger", and then to the state where the wings expand outwardly from the position of being fitted in the sides of the case body. The latter state corresponds to the state of being capable of launching (launchable state, the so-called cocking state in which the lancet can be launched or triggered). The process of shifting to the launchable state will be described in detail below.

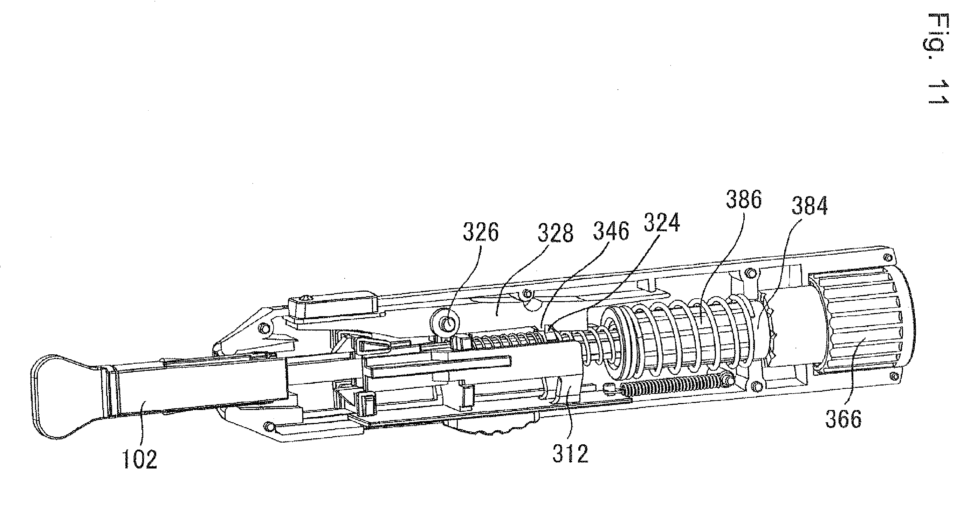

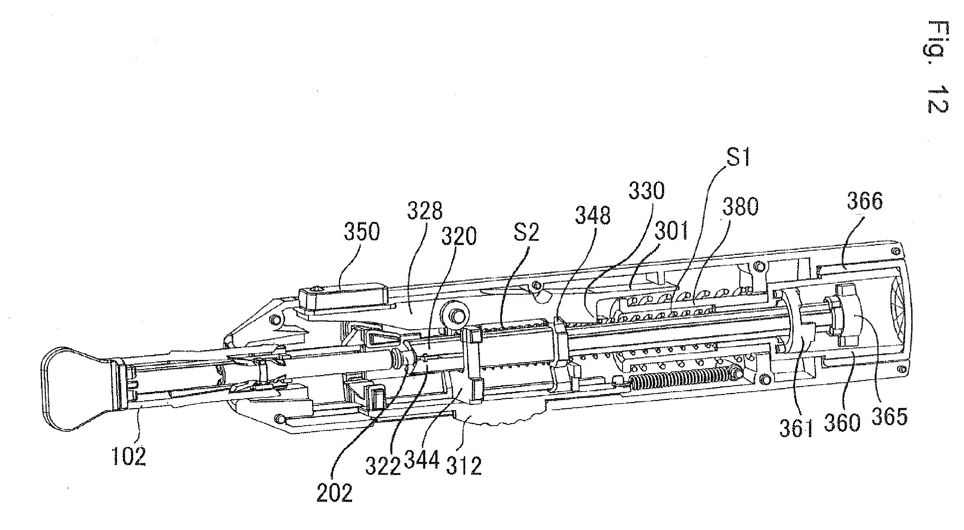

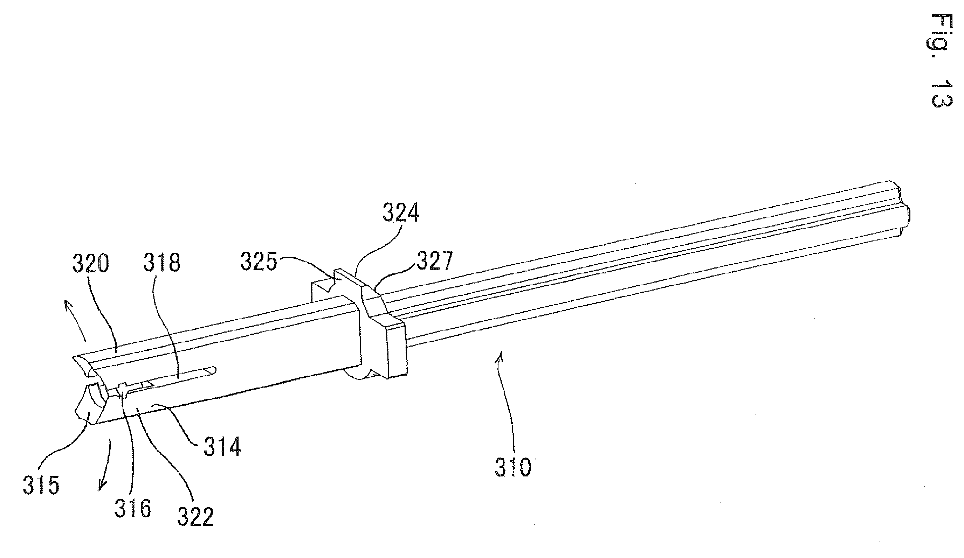

Fig. 11 and Fig. 12 are perspective views of the process of being inserting the lancet assembly 100 through the front end opening of the injector 300 in order to set the lancet assembly 100. In the embodiment illustrated, the lancet assembly that uses the lancet shown in Fig. 10 is shown as example. For the ease of understanding the relationship between the lancet assembly 100 and the injector 300 as well as the inner structure of the injector, Fig. 11 shows the state where the housing half 308 located on the near side of the injector housing is removed. Also for the purpose of illustrating the state of the lancet 200 in the lancet case 102 and the state of the plunger 310 (shown in enlarged view in Fig. 13) that launches the lancet, Fig. 12 shows the state where near side halves of the lancet case 102 is cut away and the state where a near side half of the ejector 312 is cut away. For the ease of understanding the pricking depth adjusting mechanism which will be described later, Fig. 12 shows the state where near side halves of a pricking depth adjusting drum 360, a drum rotation limiter 380 and a cap-like knob 366 which will be described later are removed.

Fig. 11 and Fig. 12 show the state immediately before the rear end portion 202 of the lancet body abuts against the seat surface 315 of the front end portion 314 when the lancet assembly 100 having the wings 116 closed (the state shown in Fig. 1) is inserted through the front end opening 302 of the injector 300 and the rear end portion 202 of the lancet body is moved toward the front end portion 314 of the plunger 310 in the injector. Then, when it is attempted to retract the rear end portion 202 further in this state, the legs 320 and 322 that constitute the front end portion 314 of the plunger are opened (upward and downward in the shown embodiment) by the rear end portion 202.

As shown in Fig. 13, the front end portion of the plunger 310 is bifurcated into two portions, and a first leg 320 and a second leg 322 that define a small gap 318 therebetween. The plunger is formed by, for example, molding a plastic material, so that the first leg 320 and the second leg 322 are capable of elastically deforming. When these legs are subjected to forces acting outwardly as indicated by the arrows, these legs deflect outward so as to expand the opening of the gap 318.

With this configuration, when the lancet assembly 100 shown in Fig. 1 is inserted into the injector 300, the rear end portion 202 of the lancet body 204 abuts against the seat surface 315 of the front end portion 314 of the plunger. An attempt of inserting the lancet assembly thereafter causes the rear end portion 202 of the lancet body 204 to apply a force of pressing backward to the seat surface 315. Since the seat surface 315 is inclined, this force also generates a force that acts in the direction indicated by the arrows in Fig. 13, so that the first leg 320 and the second leg 322 elastically splay toward the outside.

When the first leg 320 and the second leg 322 splay out as described above, the rear end portion 202 of the lancet body 204 can move further rearward, so that the bump 218 of the rear end portion 202 of the lancet body 204 fits into the recesses 316 and, at the same time, the legs that have expanded outward move to return somewhat so as to restore the original shape. As a result, the rear end portion 202 is held between the legs 320 and 322 and snugly fits into the gap 318, that is, held by the plunger. At this time, it is preferable that the surface 221 that opposes to the front side of the protruding portion 218 of the lancet body makes close contact with the seat surface 315 provided on the distal end of the plunger 310.

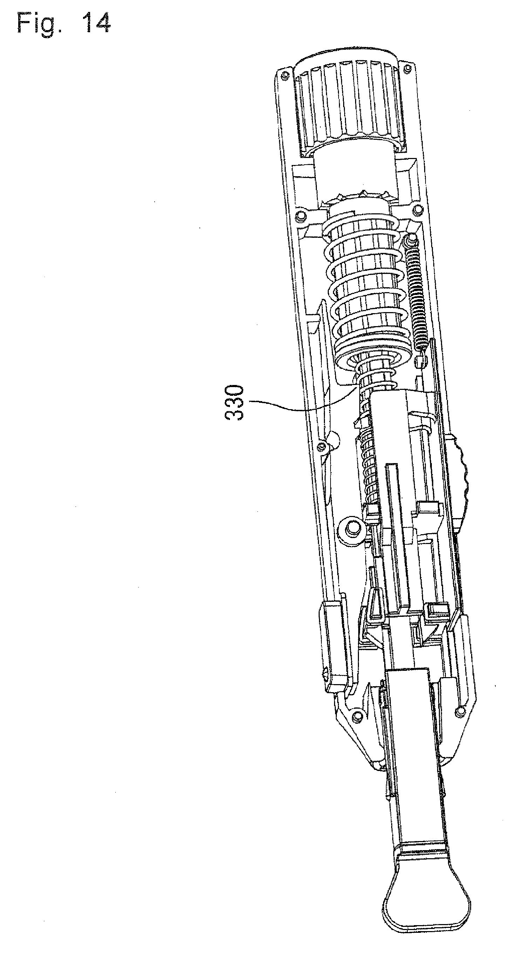

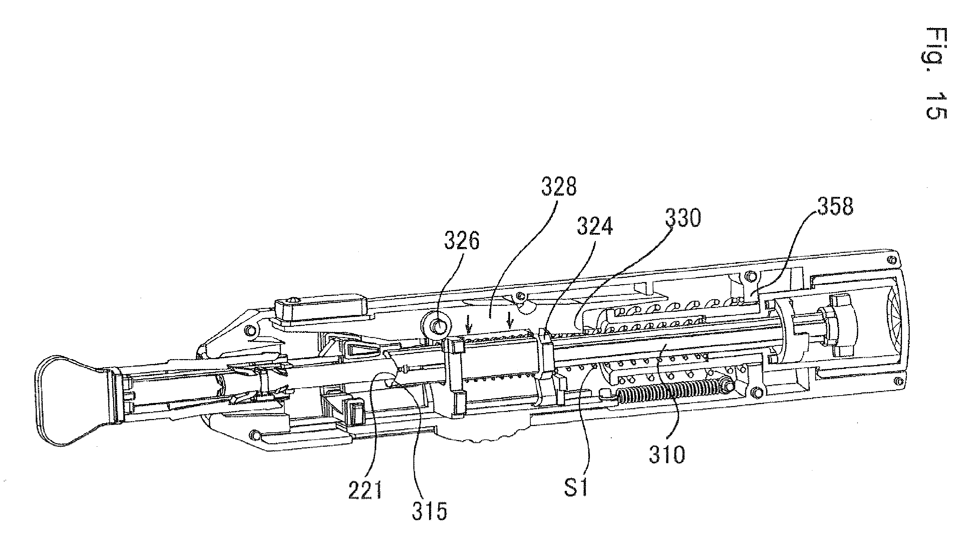

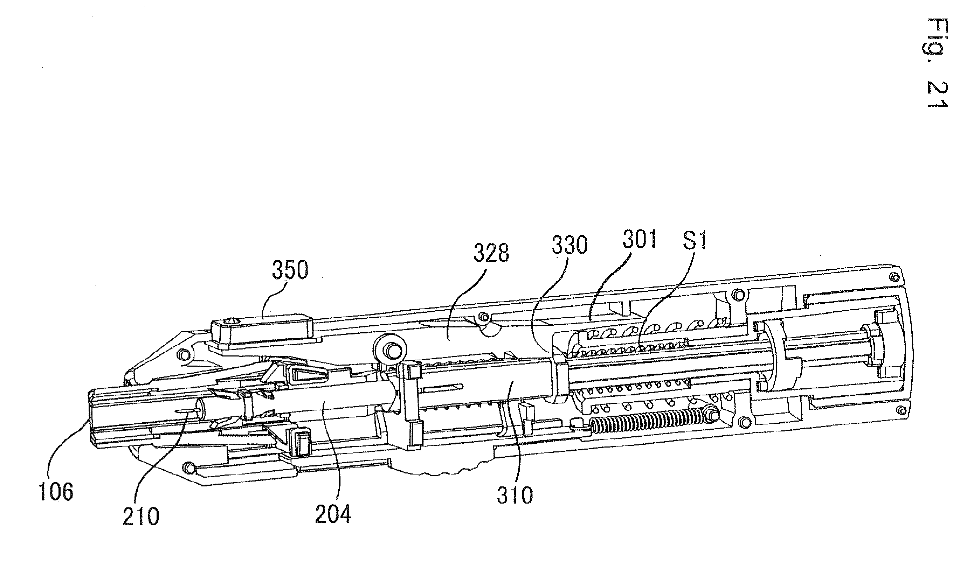

The state of the rear end portion 202 of the lancet body grasped by the plunger as described above is schematically shown in Fig. 14 and Fig. 15, similarly to Fig. 11 and Fig. 12. As will be understood from Fig. 15, the sloped surface 221 that opposes to the front side of the protruding portion 218 of the lancet body 204 abuts against the seat surface 315 provided on the distal end of the plunger 310. The plunger 310 has a launching spring S1 (see, for example, Fig. 21) disposed around the plunger 310 between the in the case where of the protruding member 324 provided around an intermediate portion of the plunger and a rear partition 358 (or a position just near side of the rear partition as illustrated).

Grasping of the rear end portion of the lancet by the front end portion of the plunger described above is preferably achieved by such a design that the rear end portion of the lancet is fitted into the front end portion of the plunger with a force smaller than the force necessary to substantially begin the compression of the launching spring S1. Specifically, the front end portion of the plunger is composed of a plurality of the legs so that, when the rear end portion of the lancet body is brought into the abutment against the distal end of the leg and then a force is applied further, the legs easily open and the protrusion of the rear end portion of the lancet fits into the recesses formed on the inside of the legs that have opened, and the legs then close due to their elasticity. The elasticity of the legs can be easily obtained by forming the plunger from a resin.

After rear end portion 202 of the lancet body has been held as described above, the lancet assembly 100 is retracted further. Then the rear end portion 202 of the lancet abutting against the front end portion 314 of the plunger forces the plunger to retract so as to compress the launching spring S1. At this time, the protrusion 324 of the plunger retracts gradually, so as to exert a force that gradually pushes upward the trigger lever pivoted by a shaft 326 against the force exerted downward by the trigger lever 328 at the lower edge of the trigger lever (as indicated by the arrows in Fig. 15) and on the rear side behind the shaft 326.

Then immediately after the gradually retracting protrusion 324 has passed below the shoulder (corner) 330 located at the rear end of the trigger lever, the shoulder 330 of the trigger lever abruptly moves downward. At this time of moving downward, the front side of the protrusion 324 is in contact or nearly in contact with the rear side of the shoulder 330. If the insertion of the lancet assembly is stopped at this time (that is, if the retraction of the plunger is stopped), the protrusion 324 of the plunger can be put into engagement with or abutment against the shoulder 330 of the trigger lever in such a state that the compressed launching spring S1 is exerting a force directed forward.

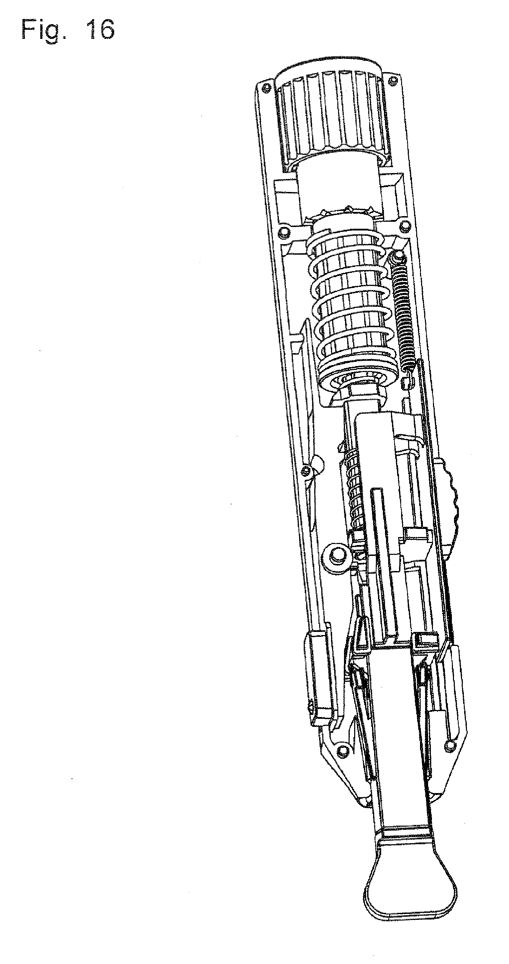

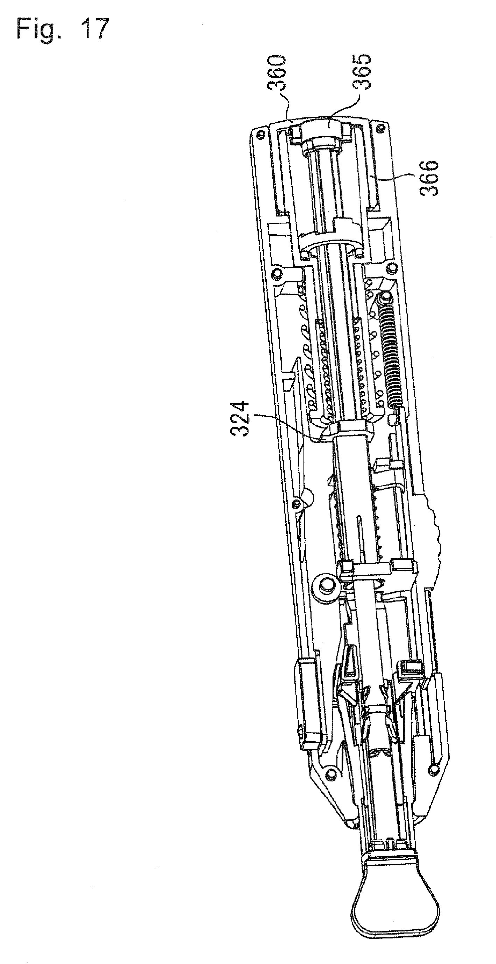

Thus the state of the rear end of the trigger lever just having moved downward may be referred to as "the state of the trigger lever being capable engaging with the plunge." The lancet assembly and the injector are preferably designed such that, when loading the lancet assembly of the present invention, after the above-mentioned state has been achieved, the insertion of the lancet assembly is additionally continued further (namely the plunger is retracted further) so as to open the wings 116 that are in the closed condition. The wings 116 are opened by the wing splaying members 342 provided in the injector. The state of the wings 116 being opened is schematically shown in Fig. 16 and Fig. 17, similarly to Fig. 11 and Fig. 12.

It is preferable to design such that the plunger does not retract further when the wings 116 are opened. For example, such a design may be employed as the hitting member 365 provided on the rear end portion 365 of the plunger abuts against the rear inside wall of the pricking depth adjusting knob (or grip) 366 that covers the drum 360 which surrounds the rear end portion and is not capable of retracting further, as will be understood for example from Fig. 17.

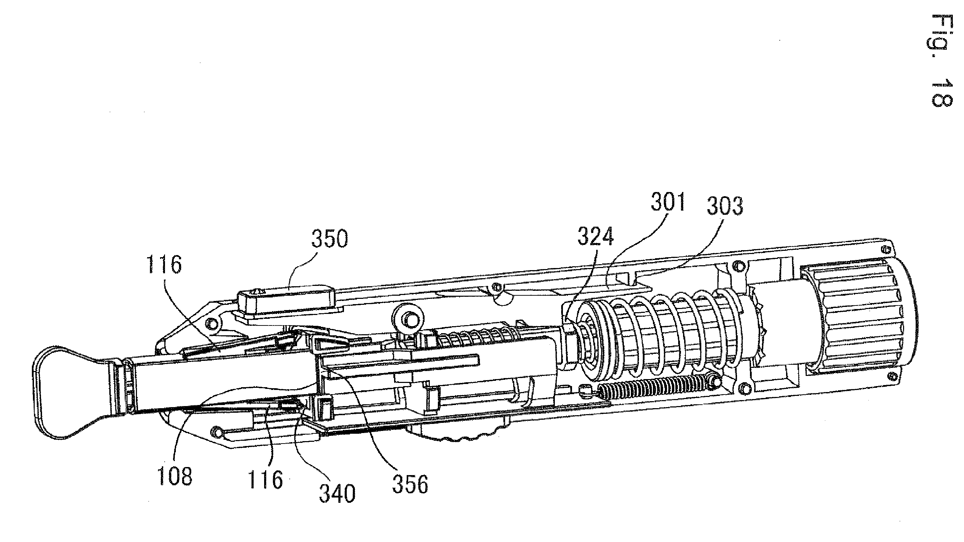

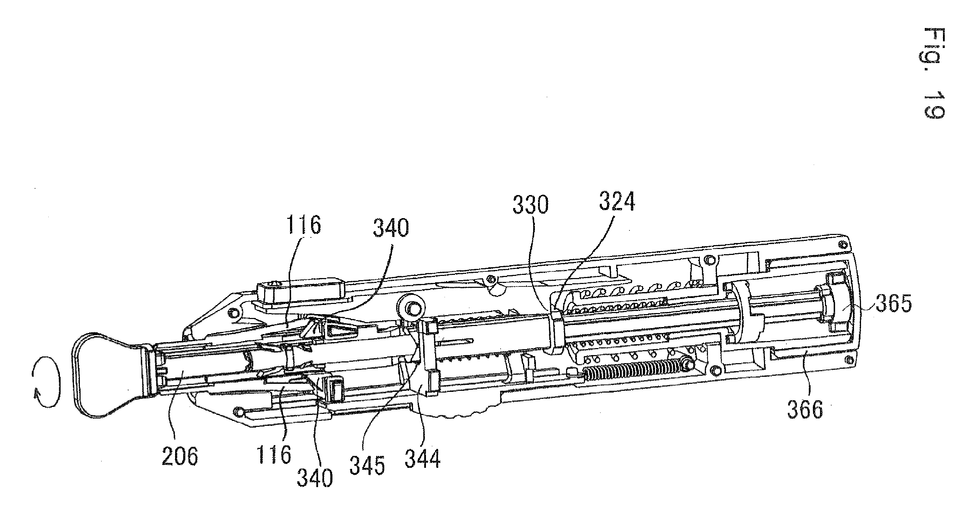

When the force applied to the lancet assembly is removed at this state, the launching spring S1 that has been compressed expands somewhat so that the plunger moves a little forward. As a result, the protrusion 324 of the plunger abuts against, namely engages with the rear end 330 of the trigger lever and "the launchable state" is established for the first time thus completing the loading of the lancet assembly in the injector. This means that, when this engagement gets undone in this state, the plunger can be caused to instantly move forward by the action of the launching spring S1. This state of the lancet being able to be launched is schematically shown in Fig. 18 and Fig. 19, similarly to Fig. 11 and Fig. 12.

Comparison of Fig. 19 with Fig. 17 shows that the hitting member 365 provided on the plunger is abutting against the rear inner wall of the knob 366 in Fig. 17, while the rear end portion 365 of plunger has moved a little forward and the in the case where of the plunger has also moved forward in Fig. 19. It is preferable to design so that, in "the launchable state", the fitting portion 217 of the lancet cap protrudes from the front end opening 106 of the case body 114 as seen from Fig. 18, and therefore it is therefore disposed at a position ahead of the front end opening of the case body 114. When the fitting portion 217 has the narrowing portion 219 as shown in Fig. 3, it is preferable that at least a portion thereof protrudes. When the narrowing portion is not provided, it is preferable that substantially whole of the fitting portion 217 protrudes (namely, no more in the fitting state).

The lancet case 102 is fitted in the injector by means of the protrusion 112 thereof and the protruding portion 304 of the injector 300 (therefore, the lancet case and the injector are attached to each other). Therefore, the lancet held by the plunger is caused to move a little forward by the small forward movement of the plunger. It is preferable that the fitting portion is caused to protrude as described above by this forward movement.

In either embodiment, it is preferable that, when the launchable state is achieved, at least a portion of the fitting portion of the lancet cap is positioned ahead of the front end opening of the case body. As a result, the function of the fitting portion to prevent the lancet cap from turning relative to the lancet body is substantially lost so as to enable the lancet cap to turn relative to the lancet body around the pricking member.

In the state where the protruding in the case where is engaged by the shoulder 330 that is located at the rear end of the trigger lever as described above, it is preferable that the gap 318 of front end portion 314 of the plunger 310 does not expand, namely the legs 320 and 322 do not deform to expand outward. In order to prevent such expansion, it is preferable that, in the state of the protruding in the case where abutting against the rear end 330 of the trigger lever as shown in Fig. 19, the front end portion 314 of the plunger is located within the opening 345 of the front partition 344 provided in the housing and the wall that defines the opening 345 thereof just surrounds the front end portion 314 of the plunger 310, so that the front end portion 314 does not expand in the vertical direction. In the state prior to grasping of the lancet body 202 by the front end portion 314 of the plunger, the front end portion 314 is located at a point ahead of the front partition 344 and is therefore capable of easily expanding (hence capable of grasping), as shown in Fig. 12.

In the embodiment illustrated, a plate-shaped member 301 extends toward the rear from the upper edge of the end surface of the shoulder 330 located at rear end of the trigger lever. The plate-shaped member 301 is adapted such that an inward force is exerted between the protrusion 303 that supports it and the shaft 326, and an outward force is exerted in the portion ahead of the shaft 326. With this configuration, the inward force is always exerted at the rear end of the trigger lever so as to achieve "the state of the trigger lever being capable of engaging with the plunger" and "the launchable state" as described above. When a button 350 is depressed inward to launch the lancet, the plate-shaped member 301 presses the protrusion 303 and undergoes elastic deformation, so that a force is exerted by the deformation to push back the button.

The above description relates to the embodiment in which the wings 116 are opened after "the state of the trigger lever being capable of engaging with the plunger" has been achieved. In another embodiment, such a design may also be employed as the wings 116 begin to open before "the state of the trigger lever being capable of engaging with the plunger" is achieved, and the wings 116 become fully open when the state "the state of the trigger lever being capable of holding (or engaging with) the plunger," has been achieved.

In the latter embodiment described above, it is also preferable that the lancet assembly is set in the injector with the plunger being retracted a little, after "the state of the trigger lever being capable of engaging with the plunger" has been achieved and, as a result, at least a portion of the fitting portion of the lancet cap protrudes from the front end opening of the case body in "the launchable state."

In a further embodiment, such a design may also be employed as the wings 116 begin to open before "the state of the trigger lever being capable of engaging with the plunger" is achieved, and the wings become fully open substantially at the same time as "the state of the trigger lever being capable of engaging with the plunger" is achieved. These various embodiments can be achieved by, for example, shifting the position of the wing splaying member forward or rearward within the injector. In other embodiment, it can be achieved also by changing the length of the wings.

According to this embodiment, upon loading the lancet assembly into the injector, when the lancet assembly is inserted through front end opening of the injector, the rear end portion of the lancet abuts against the front end portion of the plunger. When the inserting action is continued thereafter, the rear end portion of the lancet is held by the front end portion of the plunger and then the launching spring is compressed by the retraction of the plunger. The protrusion of the plunger causes the rear end of the trigger lever (or the shoulder located at the rear of the trigger lever) to move outward, that trigger lever is constituted such that a force is exerted to act inward (namely on the plunger side), to move outwardly. Then at the instant when the protrusion retracts further by getting over the rear end of the trigger lever (or the shoulder), the rear end of the trigger lever returns inward (the plunger does not retract further). As a result, the protrusion (for example, flange form) engages with or abuts against the rear end of the trigger lever (or the shoulder) while the spring remains compressed (after this state has been achieved, the plunger does not retract further, and preferably unable to retract further) and, substantially simultaneously, opening of the wings is completed.

In this case, when the rear end of the trigger lever has returned inward, the state of the trigger lever being capable of engaging with the plunger" is achieved, and at the same time, opening of the wings toward outside is completed, and therefore it is unnecessary to cause the rear end of the lancet to retract further. As a result, in this embodiment, "the launchable state" is achieved at the same time as the rear end of the trigger lever has returned inward.

Also in the above further embodiment, it is preferable that at least a portion of the fitting portion of the lancet cap protrudes from the front end opening of the case body when "the state of the trigger lever being capable of engaging with the plunger" correspondingly to "the launchable state" is achieved.

In either embodiment, the wings of the lancet assembly are in the splayed out state when the launchable state" is achieved. As a result, the abutting portions 122 provided on the inside of the wings 116 do not hamper the forward movement of the protruding portion 212 of the lancet body. That is, the lancet body is in a free state in which the restriction on the forward movement is removed. when the state of the engagement or abutment in "the launchable state" is removed by moving the rear end of the trigger lever (or the shoulder) outward after "the launchable state" has been achieved, the launching spring S1 that has been compressed expands instantly. This expansion is utilized so as to launch the lancet body which has been in the free condition as described above while the distal end portion of the pricking member is exposed, and prick the predetermined portion. When the launchable state has not been achieved, on the other hand, the abutting portions 122 provided on the inside of the wings 116 hamper the forward movement of the protruding portion of the lancet body since opening of the wings toward outside has not been completed.

In the state where the loading of the lancet assembly 100 into the injector 300 has been completed as shown in Fig. 18 and Fig. 19, what is to be most noted is that the wings 116 provided on both sides of the lancet case 102 are in the splayed out condition. The wings 116 have been splayed out because the wing splaying members 340 are provided in the injector. The wing splaying members cause the wings to splay (or open) in the state of the rear end portions of the wings being fitted in the side of the lancet case, so that the wings extend outward toward the rear obliquely with respect to the case body. The wing splaying members may have any appropriate form as long as it is capable of causing the wings to expand. In one form, the wing splaying member is a member that defines a slope in the form of a line or plane that extends obliquely with respect to the rear end of the wing that retracts upon the loading of the lancet assembly.

In one specific embodiment, the wing splaying member is in the form of a pair of wedge-shaped members provided in the injector which members are separated from each other while forming a tapered off shape (decreasing width toward the front end), preferably in the form of a pair of wedge-shaped members that define the sloped surfaces inclined inwardly toward the front end of the injector, front ends of which members can enter the inside of the rear ends of the wings fitted in the sides of the lancet case. When the lancet assembly is inserted rearward through the front end opening of the injector, the distal ends of the rear ends of the wings of the lancet case are positioned so as to come onto the front ends of the wedge-shaped sloped surfaces. As the insertion is continued, the rear ends of the wings slide over the sloped surfaces and, as a result, the force that maintains the wings in the state of folded along the case body is removed so that the wings expand outwardly and this state is maintained. Instead of the wedge-like members, plates may also be disposed obliquely so as to define the similar sloped surfaces.

In the embodiment illustrated, the wing splaying member 340 acts upon at least the rear ends (preferably only the rear ends) of the wings 116, more specifically applies an outward force to the rear end portion of the wing, thereby to expand the wings. More specifically, the wing splaying member may be a pair of wedge-like members 340. The wedge-like members 340 have a shape that tapers off (narrowing toward the front end) while departing from each other and, as a result, define the sloped surfaces 342 inclined inwardly toward the front end of the injector,

It is advantageous to provide the wing splaying member of the wedge shape since it makes possible to automatically expand the wings simply by inserting the lancet assembly rearward. When the wings have expanded in this way, loading of the lancet assembly is completed. Accordingly, it is preferable that the lancet assembly is fitted as predetermined in the injector by the above mentioned "two elements which engage or fit with each other " while the wings are expanded.

As seen from Fig. 20 that is a schematic perspective view of a half 306 of the injector housing, the wedge-like members 340 are provided to the housing half The distal end portions 341 of the wedge-like members 340 are adapted to enter the inside of the rear end portions 120 of the closed wings 116 of the lancet case 102, immediately before loading of the lancet assembly 100 into the injector 300 is completed. When the lancet assembly is retracted further thereafter, the rear end portions of the wings 116 move up while sliding along the sloped surfaces 342 of the wedge-like members 340, so that the wings 116 expand outward. Thus it is advantageous to provide the wedge-shaped members since it makes possible to automatically expand the wings 116 simply by inserting the lancet assembly rearward for setting.

Fig. 9 is a schematic perspective view showing the lancet assembly 100 when the wings 116 have expanded as described above. In Fig. 9, the lancet case 102 is shown with the surface of the near side cut away so that the state of the lancet disposed inside can be seen. As will be easily understood, the position of the abutting portion 122 has moves outwardly as the wings 116 have expanded, in comparison to the state when the wings 116 are not expanded (for example, the state shown in Fig. 2) . As a result, the abutting portion 122 cannot function as a abutting portion any more. Thus when it is attempted to move the lancet forward in the state shown in Fig. 21 where the lancet cap is removed, the protruding portion 212 of the lancet body 204 does not hit the abutting portion 122 and therefore forward movement of the lancet is not hampered.

Thereafter, the lancet cap is normally twisted off (the weakened portion is broken) to expose the distal end portion of the pricking member. Then the trigger lever is operated so as to move the plunger quickly forward, thereby launching forward the lancet body of which distal end portion s exposed and carrying out the pricking operation on the predetermined portion. At this time, the protruding portions of the lancet body passes between the pair of abutting portions 122 of the wings and moves further forward. After pricking, the lancet body quickly moves rearward and the protruding portions of the lancet body stays at a position behind the abutting portions of the wing, thus completing the pricking operation. This process will be described in more detail below.

After the loading of the lancet assembly 100 into the injector 300 has been completed as described above, the lancet cap 206 is removed. In "the launchable state", it is preferable that at least a portion of the fitting portion 217 protrudes from the front end opening of the case body as described above. The weakened portion is broken by applying forces to the lancet body 204 and the lancet cap 206 in opposite directions around the extending direction of the pricking member (as indicated by the arrow in Fig. 19) and, in addition to this or instead of this, pulling so that these members are separated away from each other along extending direction of the pricking member. Then, when the lancet cap 206 is removed away from the lancet body 204, the distal end portion of the pricking member 210 is exposed from the lancet body 204 in the lancet case as shown in Fig. 21 similarly to Fig. 12. In this way, the lancet cap performs the function of covering the distal end portion of the pricking member in advance. It is noted that it is of course that the distal end portion of the pricking member is able to be exposed by for example pulling(or pulling while twisting) the lancet cap relative to the injector when the lancet does not have the weakened portion.

In one preferred embodiment, the weakened portion of the lancet can be broken by turning (twisting) the lancet body and the lancet cap in opposite directions around the extending direction of the pricking member. In other words, the weakened portion 208 is designed so as to be broken with such a turning operation. For example, the kind of resin that constitutes the weakened portion and/or a thickness of the weakened portion is selected so as to enable such breaking. After breaking the weakened portion, the lancet cap is brought away from the lancet body, so as to expose the distal end portion of the pricking member from the lancet body.

In one preferred embodiment, in order to enable the rotation around the extending direction of the pricking member during the twisting operation described above, one of an end surface of the front end portion of the lancet body and an end surface of the rear end portion of the lancet cap which end surface opposes to the former end surface has protruding portions, and tops of such protruding portions define a hypothetical flat surface that extends at right angle to the extending direction of the pricking member, and the other end surface defines a flat surface that extends at right angle to the extending direction of the pricking member. In the embodiment illustrated (refer to, for example, Fig. 3), the rear end surface of the lancet cap has protruding portions 209 that have the tops 207, while the end surface of the lancet body that opposes to the former defines the flat surface.

The "hypothetical flat surface" described above is not an actual plane that really exists, but a single imaginary plane that abuts against the tops of the protrusions. Mathematically speaking, a flat surface can be defined by identifying three points that do not lie on one straight line. Accordingly, in the lancet of the present invention, the hypothetical surface defined by the tops of at least three protrusions is perpendicular to the extending direction of the pricking member.

In order to make it easy to twist off the lancet cap 206 by applying such force that causes the lancet body and the lancet cap to turn in the opposite directions around extending direction of the pricking member in the lancet case 102, it is preferable that the lancet body 204 is formed with its cross section of which shape makes it impossible to rotate in the lancet case, while the rear portion of the lancet cap 206 located in the lancet case has a cross section of a shape that enables it to rotate in the lancet case. For example, in the case where the lancet case that accommodates the front portion of the lancet body and the rear portion of the lancet cap has a rectangular cross section, the front portion of the lancet body may have a rectangular cross section that makes it impossible to rotate therein and the rear portion of the lancet cap may have a circular cross section.

More specifically, with considering a cross section perpendicular to the extending direction of the pricking member, the lancet body and the lancet case are constituted such that at least a portion of the wall that defines the inner space of the lancet case is disposed inside the outermost of the trajectories of the lancet body profiles formed by rotating the lancet body around extending direction of the pricking member.

In this case, it is preferable that the lancet case is not formed in a shape symmetrical with respect to the extending direction of the pricking member. In other words, the lancet case is formed in a shape that makes at least a portion of the lancet body impossible to rotate in the lancet case around the extending direction of the pricking member. For example, such a design is employed as a portion of the lancet body that is preferably proximate to the lancet cap, namely the front portion of the lancet body is formed having an oval (or rectangle) cross section perpendicular to the extending direction of the pricking member, and a portion of the lancet case has a cross section (cross section perpendicular to the extending direction of the pricking member) of such a shape that circumscribes the cross section of the lancet body or encircles the cross section of the lancet body with a small gap between the both cross sections (for example, other rectangle that encloses said oval shape (or rectangle shape), other oval located around said rectangle (or oval)or the like).

In contrast, such a design is preferably employed as a portion of the lancet cap that is preferably proximate to the lancet body, namely the rear portion of the lancet cap is formed in such a shape that allows it to rotate around the extending direction of the pricking member. Specifically, a portion of the lancet case is formed in such a shape as an inner wall that defines the inner space of the lancet case is disposed outside of the outermost of a trajectory of said portion of the lancet cap formed by rotating the lancet cap around the extending direction of the pricking member. In this case, it is preferable that said portion of the lancet cap is formed in an axial symmetry shape with respect to extending direction of the pricking member such as a cylinder or a prism, so that said portion of the lancet cap can rotate in the inner space of the lancet case. More specifically, said portion of the lancet cap is formed in a cylinder form or a polygonal prism form, and said portion of the lancet case has such a shape as the cylinder or polygonal prism can rotate inside thereof (for example, a circular cylinder or other shaped cylinder that surrounds the cylinder or polygonal prism).

It is preferable that the inner space of the case body has a cross section perpendicular to the extending direction of the pricking member which cross section does not change significantly, and it is more preferable that the cross section does not substantially change. For example, the cross section of the inner space of the lancet case is formed in a rectangular shape. In this case, for example, the lancet body is formed to have a cross section of other shape (for example, rectangle, square or oval) that cannot rotate within the rectangular inner space, and the rear portion of the lancet cap is formed to have a circular cross section that enables it to rotate within the rectangular inner space.

The lancet comprising the lancet body, the lancet cap and the weakened portion provided therebetween is preferably manufactured by molding a resin with the pricking member being inserted therein (so-called insert molding process) as mentioned previously. This method is advantageous in that it is possible to easily manufacture the lancet in large numbers that comprises the lancet body, and the lancet cap that are integrally connected via the weakened portion while the pricking member extends in these members straddling therebetween. The weakened portion is preferably formed by making the thickness smaller in the portion that constitutes the weakened portion among the body of resin that surrounds the pricking member, for example by forming a notch therein. In another preferable embodiment, the weakened portion can be formed by forming an incision in the resin layer (between the lancet body and the lancet cap) that does not reach the pricking member after forming the resin layer that surrounds the pricking member. As to the lancet without the weakened portion, it is preferably produced by the injection molding similarly.

As described above, the launching spring is kept in the compressed state when the in the case where of the plunger engages with the shoulder 330 of the trigger lever. Accordingly, in the state shown in Fig. 21, the predetermined portion (for example, a finger tip) to be pricked is applied to the front end opening 106 of the case body 114, then the trigger lever 328 is operated thereby to cancel the engaged state of the shoulder 330. This causes the launching spring S1 that has been compressed expands instantly. As a result, the plunger 310 moves forward so that the lancet body 204 of which distal end portion of the pricking member is exposed moves instantly forward and protrudes out of the front end opening 106 of the case body 114, thereby pricking the predetermined portion applied thereto.

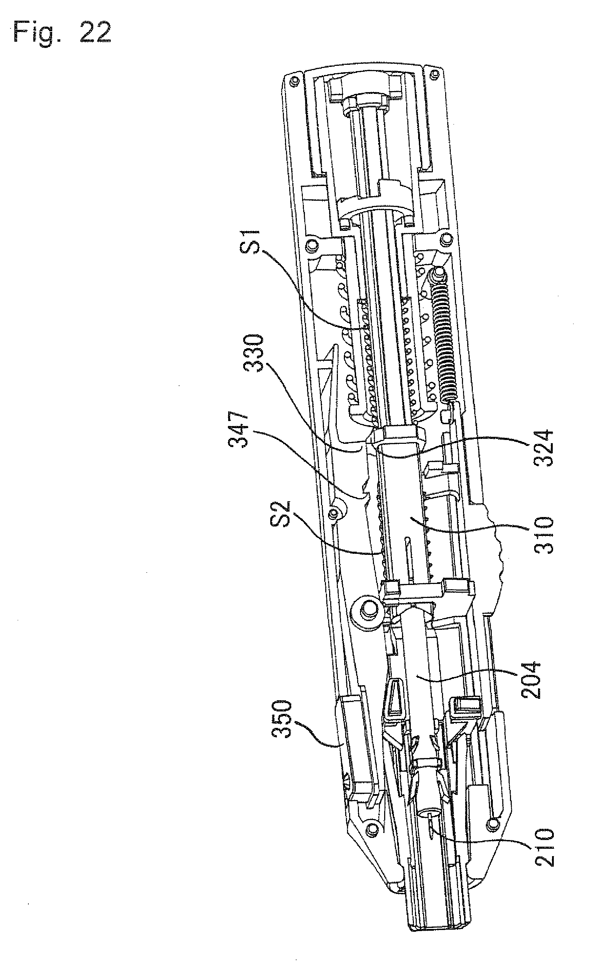

It is noted that the engaged state can be canceled by depressing the push button 350 inward, which is provided at the front end of the trigger lever. The trigger lever can rotate around a rotary shaft 326. It is preferable to provide a plate-like member 301 that extends from the outer edge of the rear end, such that the plate-like member exerts outward forces on the front portion from the push button and the portion of the trigger lever ahead of the shaft when there is no external force exerted, and an inward forces are exerted on the rear portion behind the shaft of the trigger lever. The state of the push button 350 having been depressed is schematically shown in Fig. 22, similarly to Fig. 12.

In the state shown in Fig. 22, the rear end 330 of the trigger lever moves upward and, as a result, the engagement between the rear end 330 of the plunger and the in the case where of the plunger is lost. Therefore, the launching spring S1 that has been held in the compressed state can expand instantly, so that the plunger 310, and therefore the lancet body 204 of which distal end portion of the pricking member 210 is exposed can quickly move forward.

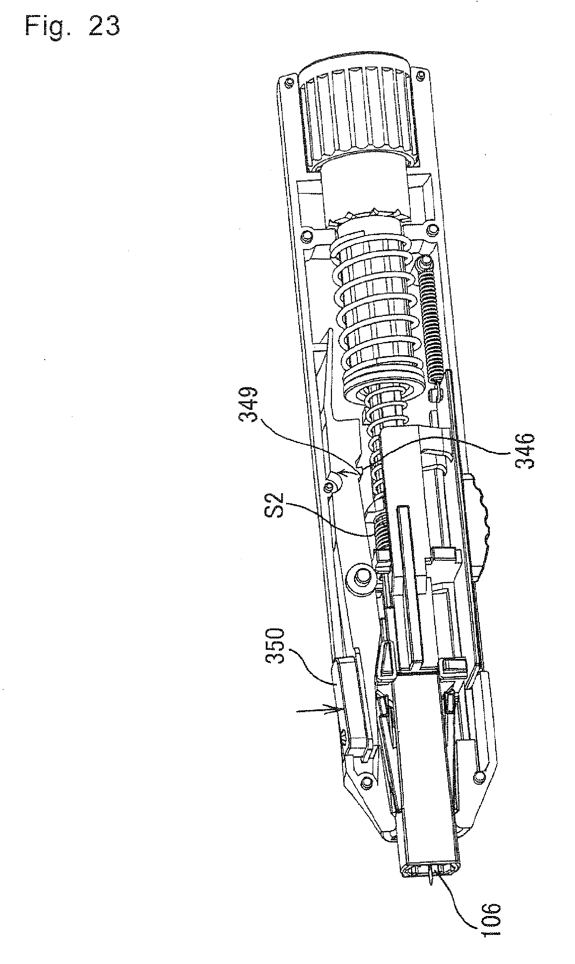

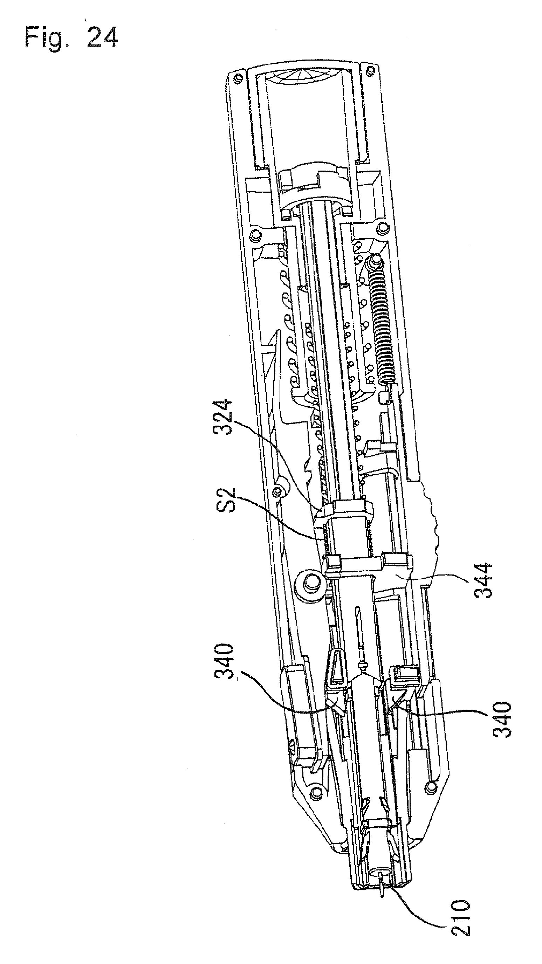

The state at the instant when the distal end portion of the pricking member 210 is ejected from the front end opening 106 of the case body 114 is shown in Fig. 23 and Fig. 24, similarly to Fig. 11 and Fig. 12. As will be understood from Fig. 9 and Fig. 24, since the wings 116 are held in the state of being expanded outward by the wedge-like members 340, the protruding portions 212 of the lancet body 204 is not blocked by the abutting portions 122 and has moved beyond it forward.

A return spring S2 is provided between the in the case where of the plunger 310 and the front partition 344 provided on the inside of the housing. As can be seen by comparing Fig. 22 with Fig. 24, the spring S2 is compressed by the forward movement of the plunger 310 when the lancet is launched. Thus the spring S2 becomes fully compressed at the instant when the distal end portion of the pricking member 210 has protruded from the front end opening 106 of the case body 114 and pricking has finished, or at a time a little before or after such instant, and then expands toward its original shape. In this way, the lancet body 204 retracts after the pricking operation.

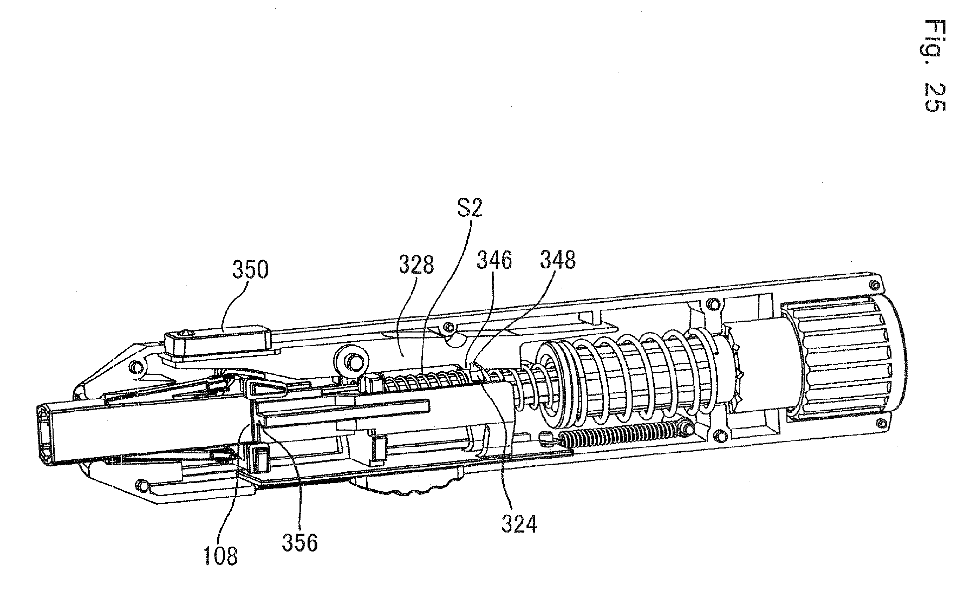

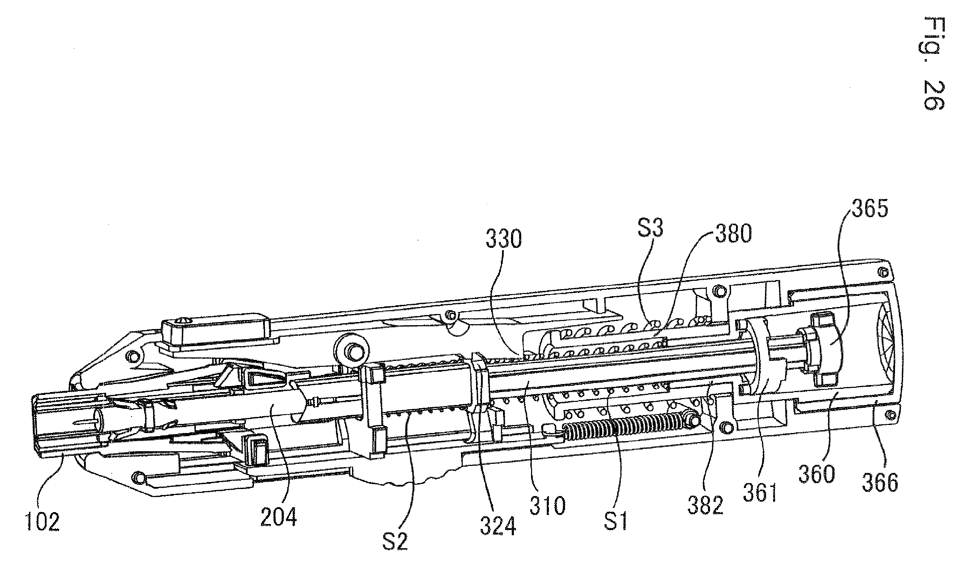

The state of the lancet body 204 retracting from the state shown in Fig. 23 and Fig. 24 is schematically shown in Fig. 25 and Fig. 26, similarly to Fig. 11 and Fig. 12. In the state shown in Fig. 25, the protruding in the case where of the plunger is caused to retract by the expansion of the return spring S2 and gets over the small protrusion 346 provided on the inner edge of the trigger lever and is fitted in the recess 348 formed behind thereof. The protrusion 346 and the recess 348 provided in this way reduce the strength of the retracting motion of the plunger. At the same time, even when the return spring S2 oscillates while contracting after expanding, the protruding in the case where of the plunger does not move forward by getting over the protrusion 346 of the trigger lever, thereby reducing the chance of accidentally repeating the pricking operations.

In such a state as the protruding in the case where of the plunger is fitted in the recess 348 located on the inner edge of the trigger lever (the edge on the side opposite to the side where the push button 350 is provided in the embodiment illustrated) as described above, the protrusion 346 disposed ahead of the recess 348 is constituted such that the engagement would not be canceled even when the plunger 310 receives a force acting forward. Specifically, a rear side surface 347 (refer to Fig. 22) that defines the protrusion 346 extends in a direction perpendicular to the pricking direction or near such direction while the front side surface that defines the protrusion 346 (see Fig. 22) defines a sloped surface that extends obliquely with respect to the pricking direction. When the trigger button 350 is depressed as indicated by the arrow to launch the lancet as shown in Fig. 23, the trigger lever rotates around the shaft 326 and the protrusion 346 moves upward as indicated by the arrow.

As a result, the shoulder 346 does not lie on the trajectory of the forward movement of the protruding in the case where of the plunger, and therefore does not prevent the plunger from moving forward. The trigger lever is constituted so as to restore the initial state when the force depressing the trigger button 350 is removed.

When such a trigger lever is used, the relationship of the protruding in the case where of the plunger being fitted in the recess 348 of the trigger lever can be maintained even when the lancet case 102 is subjected to a force acting forward by the ejector and the force eventually acts also on the plunger upon discharging the lancet assembly from the injector as will be described later. In order to make sure of this relationship, the front side surface 325 that defines the protruding in the case where of the plunger extends in a direction perpendicular to the moving direction of the plunger or at an angle near such direction as shown in Fig. 13, and the rear side surface 327 extends obliquely and forms a slope.

The perpendicular front side surface and the oblique rear side surface that define the protruding portion 324 the plunger, and the oblique front side surface and the perpendicular rear side surface that define the protrusion 346 disposed on the inner side of the trigger lever described above are combined so that protruding portion 324 of the plunger can easily get over the protrusion 346 of the trigger lever and move rearward. However, once get over thereafter, the protruding portion of the plunger cannot easily get over the shoulder 346 to move forward. In fact, at least one of the shoulder 346 and the protruding portion 324 must be deformed or broken in order to get over.

Thus in one embodiment of the injector of the present invention, the trigger lever has a bump or a recess provided ahead of the rear end (or the shoulder) on the inner side thereof, and the protrusion of the plunger abuts against the rear end of the bump of the trigger lever or is fitted in the recess before loading the lancet assembly or after launching the lancet. In other embodiment, the trigger lever may have both of a bump and a recess disposed adjacent to each other as shown and, in this case, the protrusion of the plunger fits in the recess while abutting against with the bump. In the state where the plunger is fitted in, the plunger cannot move even when a force is exerted in the direction of moving the plunger forward. However, when a force is exerted in the direction of moving the plunger rearward (such as when the lancet assembly is being loaded), it is necessary that the rearward movement is not hampered. For this reason, it is preferable that both the front side surface that defines the bump and the rear side surface that defines the recess define slopes that extend from the trigger lever obliquely rearward, as illustrated.

Fig. 25 and Fig. 26 show the state of having completed of the pricking operation. Thereafter, it is necessary to discharge the lancet assembly that has been used from the injector and properly dispose of the lancet assembly. To discharge the lancet assembly that has been used, an inward force is exerted onto the wings 116 that are in the splayed out condition in such a state as the protruding portions 212 of the lancet body are located behind the abutting portions 122 of the wings 116, so as to close and place the wings 116 along the sides of the case body thereby to confine the protruding portions 212 of the lancet body between the abutting portions 122 of the wings and the rear end opening 107 of the case body. In this state, the protruding portions 212 of the lancet body is capable of moving again only between the abutting portion 122 of the wing and the rear end opening 107 of the case body. The case body and the lancet body are designed such that the pricking member 210 with the distal end thereof exposed is disposed at a distance sufficiently away back from the front end opening 106 of the case body in such a state as the protruding portion 212 of the lancet body abuts against the abutting portion 122 of the wing. With this constitution, accidental contact with the distal end portion of the pricking member through the front end opening of the case body is substantially eliminated with the lancet assembly that has been used in which the wings are closed, and therefore the lancet assembly that has been used can be safely disposed of.

In one preferable embodiment, the abutting portion of the wing as described above has a form of a protruding portion provided between the front end and the rear end of the wing. In this embodiment, the rear side of the abutting portion of the wing can abut against the front side of the protruding portion of the lancet body and therefore the forward movement thereof can be restricted, when the lancet is inserted into the lancet case and the wings are put into the position along the side of the case body after moving the lancet body rearward relative to the lancet case so as to move the protruding portion of the lancet body to a position behind the abutting portion.

In other preferable embodiment, the abutting portion of the wing as described above defines a step (or shoulder) provided between the front end portion and the rear end portion of the wing, more specifically, the inner wall that defines the wing defines a step h (refer to Fig. 4). In this embodiment, the rear side of the abutting portion of the wing is a surface which defines the height of the step when the lancet is inserted into the lancet case and the wings are put into the position along the sides of the case body after moving the lancet body rearward relative to the lancet case and moving the protruding portion of the lancet body to a position behind the abutting portion. This surface can abut against the front side of the protruding portion of the lancet body and therefore can restrict the forward movement.

The lancet assembly that has been used is discharged from the injector in the following procedure. First, in the state shown in Fig. 25 and Fig. 26, the lancet case 102 is moved forward relative to the lancet body 204, so that the stopper 213 comes out of the rear end opening of the case body. Then the rear end portion 302 of the lancet body that is grasped is released from the plunger 310. Then the lancet assembly composed of the lancet body and the lancet case is discharged from the injector through the front end opening with the wings being closed.

The stopper 213 is provided on the lancet body for the purpose of preventing the lancet assembly that has been used from being accidentally reused. As will be understood from the foregoing description of the stopper, once the stopper of the lancet body has got out of the rear end opening 107 by moving the lancet case forward relative to the lancet body for closing the wings after the pricking operation, the lancet body cannot enter the lancet case again.

The lancet assembly and the injector are designed so that the inward force closes the wings are automatically applied when the lancet assembly that has been used is discharged from the injector. By such design, a risk related to the distal end portion of the pricking member is substantially eliminated in the lancet assembly that has been discharged. Preferably, a force is applied such that the wall defining the front end opening of the injector when the wing passes through the opening as described below. For example, a distance between partial regions (for example, two points which are opposing to each other) of a profile of the front end opening, particularly a profile of the opening to which the wings are located most adjacently upon discharging is substantially the same as, or a little larger than a distance between the outsides of the pair of the wings, inward forces are applied to the wings by the opening upon passing through the opening, so that the wings are closed.

In one embodiment, it is preferable that the injector further comprises an ejector that is capable of applying a force acting forward on the lancet case that has been inserted. By moving such an ejector forward, a force can be applied to the rear end portion of the lancet case (for example, in the state where the bump 112 disposed on the outside behind the front end of the lancet case 102 has gotten over the bump 304 located on the inside behind the front end opening of the injector and fitted in the injector).

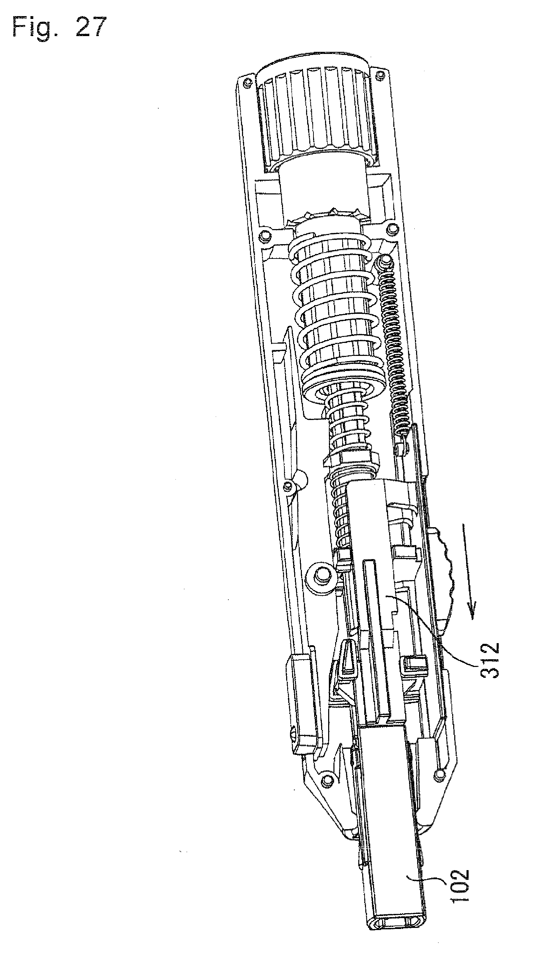

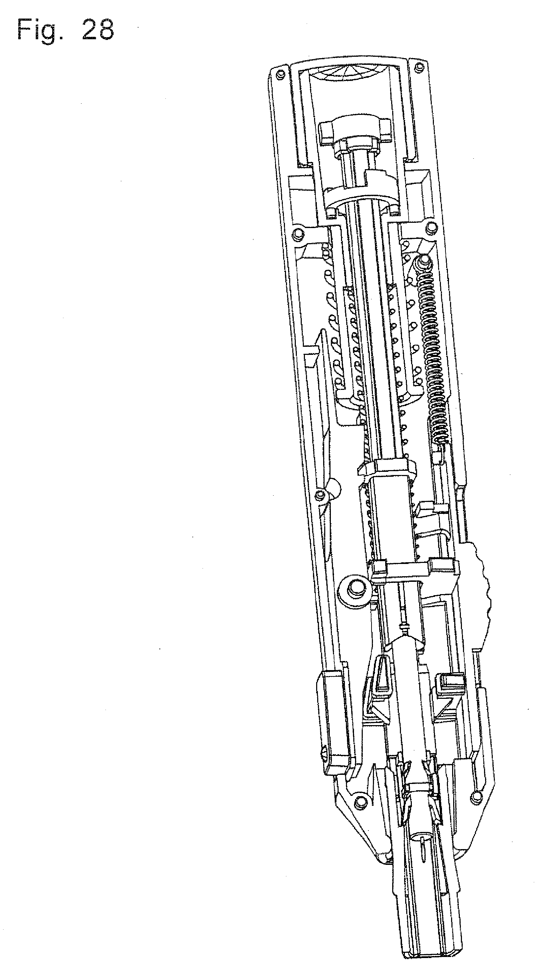

Fig. 27 through Fig. 34 schematically show the procedure of discharging the lancet assembly that has been used from the injector in sequence. The state of the ejector 312 starting to push out the lancet case 102 is schematically shown in Fig. 27 and Fig. 28, similarly to Fig. 11 and Fig. 12. Fig. 35 is a schematic perspective view of such ejector. The ejector 312 comprises a base plate 351 and a pusher 352, and is disposed in a lower portion of the injector housing 309. The base plate 351 can be moved forward within the injector 300 by sliding the operation button 354 provided on the lower side of the base plate 351 from the outside of the injector in the direction of the arrow (refer to Fig. 27). For example, such a constitution may be employed as a guide channel 355 (refer to Fig. 20) is provided on the inside of the injector housing and a rail is provided on the outside of the base plate for moving in the channel. In other embodiment, an edge 353 on the side of the base plate 351 functions as a rail that is fitted in the channel 355 to make sliding motion.

The plunger 352 is configured so that the front end portion 356 abuts or generally abuts against the rear end portion 108 of the lancet case 102 at the time when loading the lancet assembly 100 in the injector 300 is completed. The front end 356 applies a forward force as an acting member to press the rear end portion of the lancet case. Fig. 18 shows the ejector having such a configuration. In other embodiment, the plunger 352 may be so positioned as to be spaced from the rear end portion 108 of the lancet case 102, preferably spaced to an extent of generally abutting against the rear end portion 108, at the time when loading the lancet assembly 100 in the injector 300 is completed. The pusher 352 is constituted so as to be capable of pushing forward the rear end portion 108 of the lancet body 104 of the lancet case 102, preferably the opposing side face to the pusher, more preferably only the rear end surface of the side surface where the wing is absent.

In the case a force is exerted on the rear end portion of the lancet case as described above when the lancet body is grasped by the plunger after the pricking operation, the bump 112 located on the outside behind the front end portion of the lancet cap that has gotten over the bump 304 of the front end opening of the injector and fitted in the injector gets over the bump 304 of the front end opening of the injector, so that the lancet case 102 moves forward relative to the plunger 310, and therefore relative to the lancet body 204 that is grasped by the plunger.

That is, when the state shown in Fig. 25 and Fig. 26 has been achieved upon the completion of pricking, the button 354 of the ejector is operated to slide forward so as to push the rear end portion 108 of the lancet case 102 forward by the pusher 356. When the force that pushes forward exceeds a threshold that represents a force required to drive the protruding portion 112 of the lancet case to get over the protrusion 304 provided on the inside of the front end opening 302 of the injector 300, the lancet case 102 moves forward.

As described above, the forward movement of the plunger is blocked by the protrusion 346 of the trigger lever and the recess 348. As a result, the force applied by the ejector is used first for moving the lancet case 102 forward. At this time, since the wings 116 move away from the wing splaying members 340 and also the lancet case is passing through the front end opening of the injector, the wings 116 are fitted into the sides of the lancet case in turn.

Then, when the ejector is caused to slide further forward, the stoppers 213 (specifically the base portions thereof) of the lancet body abuts against the inside of the wall that defines the rear end opening 107 of the case body. When the force is increased, the wall that defines the rear end opening 107 of the case body gets over the stoppers 213 due to the shape and elasticity of the stoppers 213. As a result, the stopper 213 protrudes from the rear end opening 107 to the outside.

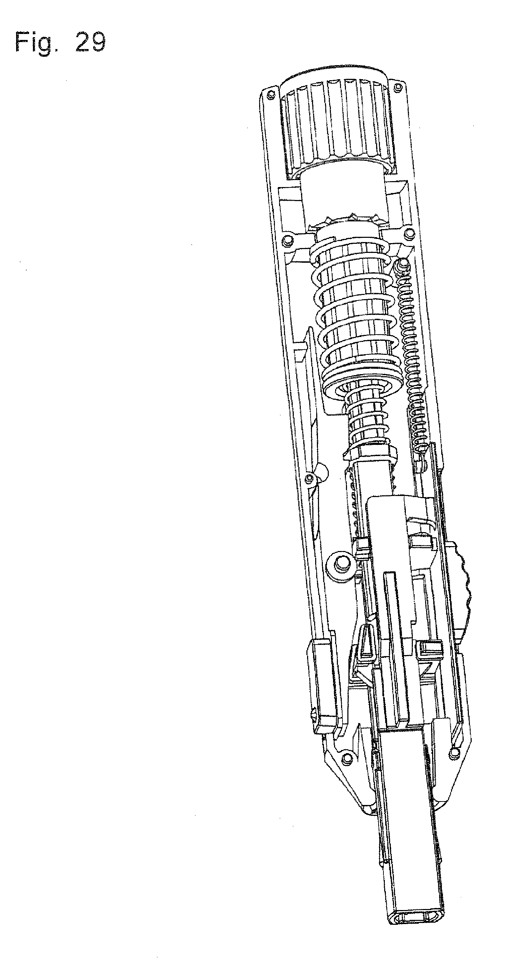

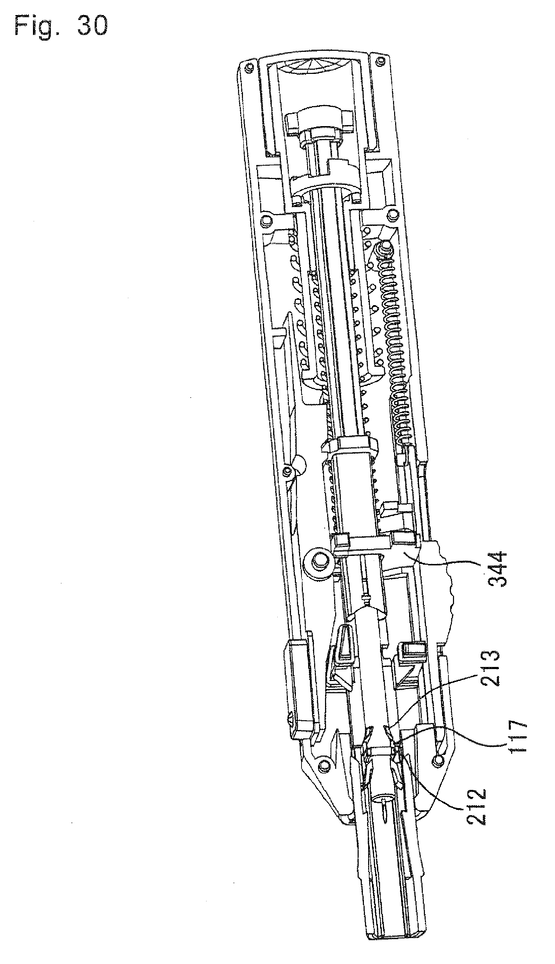

The state of the stopper 213 having got to the outside is shown in Fig. 29 and Fig. 30, similarly to Fig. 11 and Fig. 12. As shown in Fig. 30, the stoppers 213 are disposed adjacent to the right-hand side of the wall 217 that defines the rear end opening 107 of the case body 114.

As described above, when the lancet case 102 moves forward, each wing splaying member 342 does not act on the rear end portion 120 of the wing 1116, followed by the stoppers 213 of the lancet case getting out through the rear end opening 107 of the case body 114. Further, upon gradually getting out of the lancet case 102 while passing through front end opening of the injector, the profile of the front end opening 302 fits the wings 116 into the sides of the lancet case from their front portions in turn.

In order to be fitted into as described above, at least portion profiles of wall portions just beside of which the wings of the lancet case pass of the profile of the wall which defines the front end opening of the injector have to correspond to profiles of side surface of the lancet case which is in the state wherein the wings are fitted into the sides of the case body. That is, the wall portion of the injector which is opposing to the wing has a shape that allows it to contact with the wing of the lancet case which is getting out so as to apply the inward force to the wing. Therefore, the such wall portion is complementary to the side surface of the lancet case in the state wherein the wings are closed. Of course, the distance between thus opposing wall portions is substantially equal to the distance between the side surfaces of the lancet case in the state wherein the wings are closed.

As a result, the wings splaying outward are fitted into the sides of the case body by the application of the inward forces to the wings by means of the front end opening of the injector without being disturbed by the wing splaying members. Then, the lancet assembly which has been inserted into the injector is discharged by the ejector in the state wherein the wings have been fitted in the sides.

Then, when the ejector is caused to slide further forward, the wall 117 that defines the rear end opening 107 of the case body 114 abuts against the protruding portions 212 located ahead of the stoppers 213. This protruding portions are harder than the stoppers213, and do not have the flexibility of the stoppers 212. As a result, the wall that defines the rear end opening 107 cannot get over the protruding portions 212. That is, the protruding portions 212, unlike the stopper 213, are not capable of getting out of the lancet case. The state of the protruding portions of the lancet body making contact with the wall 217 that defines the rear end opening 107 is also shown in Fig. 29 and Fig. 30.

In the embodiment illustrated, since the stoppers 213 and the protruding portions 212 are located at a small distance from each other, the ejection of the stopper 213 to the outside and the abutment of the protrusion against the wall 217 that defines the rear end opening of the protruding portion 212 take place substantially at the same time.

As will be understood from Fig. 30, when the protruding in the case where of the plunger is engaged with (or abuts against) and/or fitted in the protrusion 346 and/or the recess 348 located on the lower side of the trigger lever, the front partition 344 is located at a position behind the legs 320 and 322 of the plunger (in the embodiment illustrated) and, as a result, the front partition 344 does not hamper the elastic expansion of the distal ends of the legs. It is noted that both of the protrusion and the recess are present, these are preferably adjacent to each other as shown.

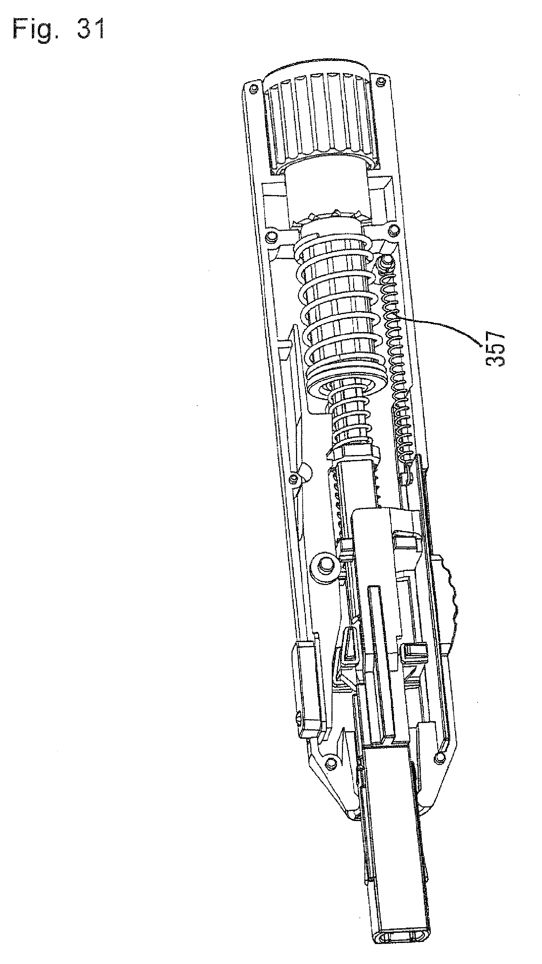

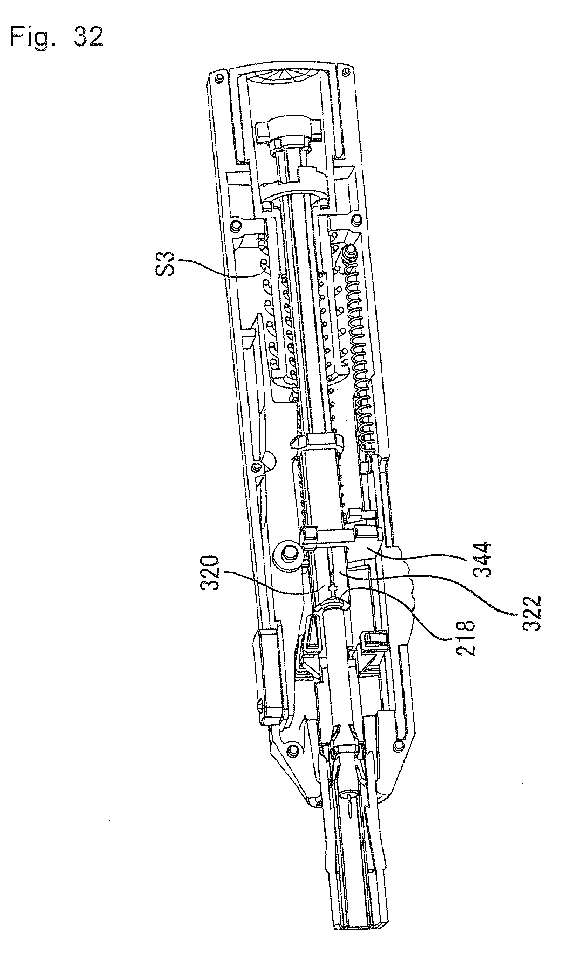

When a force is applied so as to cause the ejector to slide further forward in the state described above, the plunger cannot move forward because of the fitting described above, although the force that acts forward is transmitted to the protruding portion 218 of the lancet body grasped by the legs 320 and 322 of the plunger 310. As a result, the front side surface 231 (refer to Fig. 3) that inclines inwardly toward the front of the protruding portion 218 of the lancet body acts, due to the configuration thereof, to expand the legs. When such a force directed forward exceeds a threshold, the legs elastically expand outward so as to release the protruding portion 218 and the rear end portion 202 of the lancet body 204 that have been grasped. The state after the plunger has just released the protruding portion 218 disposed on the rear end portion 202 of the lancet body is shown in Fig. 31 and Fig. 32, similarly to Fig. 11 and Fig. 12.

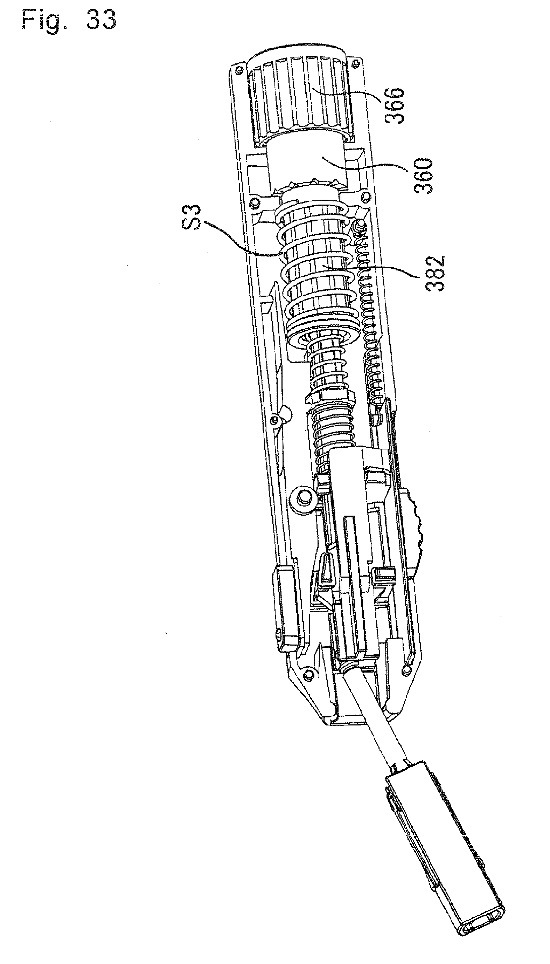

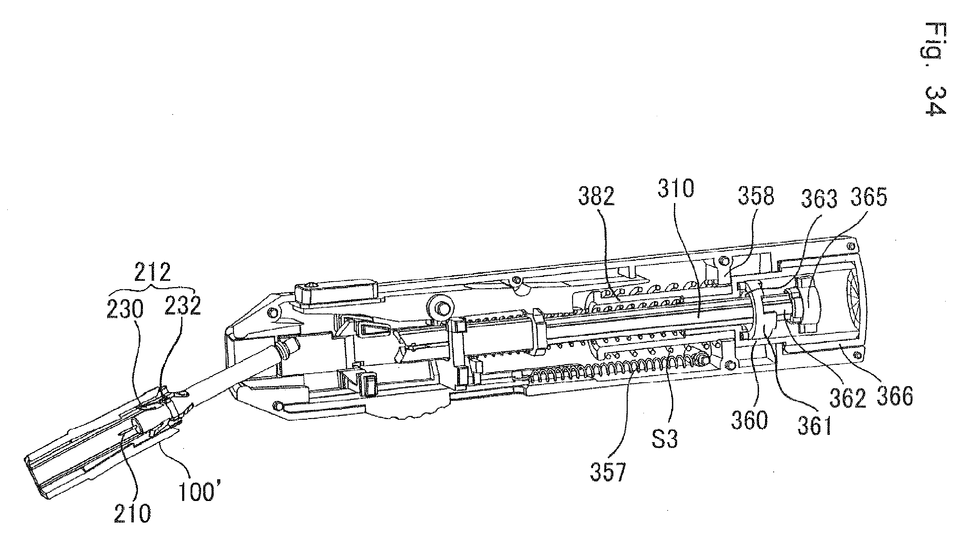

Then, when the ejector is caused to slide further forward, the lancet case that includes the lancet body separated from the plunger is discharged through the front end opening of the injector to the outside. The discharging operation is shown in Fig. 33 and Fig. 34, similarly to Fig. 11 and Fig. 12. As described above, when the lancet case passes through the front end opening of the injector, the wings are fitted into the sides of the lancet case. In the case wherein only the rear end portions of the wings are substantially fitted into the sides of the lancet case so that the whole of the wings are fitted therein (for example, in the case wherein the rear end portions have triangle wing portions as shown), when the rear end portions of the lancet case pass through the front end opening of the injector, such rear end portions are configured to be fitted into the sides. It is preferable to provide the return spring 357 between the ejector and the lancet housing as illustrated, so that releasing the button 354 in the state shown in Fig. 34 causes the ejector to return to its initial position.

When the lancet case 102 moves forward, the rear end portions 120 of the wings 116 departs from the wedge member 342, and the wings 116 undergo elastic deformation so as to be positioned on the sides of the lancet case, assuming the state shown in Fig. 36 that illustrates the lancet assembly 100' of Fig. 34 or the state shown in Fig. 2 (provided that distal end portion of the pricking member 210 is exposed). In this state, as will be easily understood, the abutting portion 122 provided on the inside of the wing 116 serves the function as the abutting portion again. That is, in the embodiment shown in Fig. 36, the protruding members 230 of the lancet body 204 cannot get over the abutting portions 122 so as to move forward in the lancet case, and the protruding member 232 abut against the wall 117 that defines the rear end opening 107 of the case body 114 and cannot move further rearward. In the embodiment shown in Fig. 2, the protruding portions 212 of the lancet body 204 cannot get over the abutting portion 122 to move forward in the lancet case, and is abutting against the wall that defines the rear end opening 107 of the lancet body 114 and cannot move further rearward.

Thus since the lancet body 204 with the distal end portion of the pricking member 210 being exposed is prevented by the abutting portion 122 from moving forward, the distal end portion of the pricking member is prevented from moving forward beyond a position far inward from the front end opening 106 of the case body 114. It needs not to say that the protruding portions 212 of the lancet body 204 or the protruding members 232 are also not capable of getting rearward out of the rear end opening 107 of the case body 114. In this way, when the lancet case 102 moves forward and the wings 116 are fitted in the lancet case, the lancet body is held within the lancet case 102 with the distal end portion of the pricking member located at a position sufficiently rearward from the front end opening 106.

As will be easily understood from Fig. 36, the distal end portion of the pricking member 210 can be kept sufficiently away from the front end opening 106 of the case body 114 even when the lancet body 204 has moved to a position farthest forward in the lancet case 102, by proper choice of the sizes of the lancet case and the lancet body. As a result, the danger related to the distal end portion of the pricking member that is exposed is greatly reduced when handling the lancet case 102 in the state shown in Fig. 36.

It is preferable that the lancet case and the lancet body have means of making it smoother for the lancet body 204 to move in the lancet case 102 during pricking. The means may be, for example, the protruding member 215 of the lancet body described above. The channel 135 is defined by a pair of rails 140 provided on the inside of the side surface of the lancet case 102 so as to sandwich such protruding portion described above. Guiding means that makes it smoother for the lancet body 204 to move in the lancet case 102 can be constituted by combining such a protruding portion described above and the rails.

The lancet assembly and the injector of the present invention described above are formed from resin except for the pricking member and the springs, and particularly preferably formed by molding of resin. In addition to the ease of manufacturing, elasticity of resin can be preferably utilized in the function of the wings, the function of the stopper, a function of the plunger legs, and the fitting operation when loading the lancet assembly into the injector (for example, the relationship between the protruding portion of the lancet case 112 and the bump 304 at the front end opening 302 of the injector).

It is preferable that the injector has a mechanism that controls the depth of pricking. The injector of the present invention has a pricking depth adjusting drum disposed behind and adjacent to the rear partition in the injector housing. The plunger extends and terminates within the drum. The drum has a ring-shaped member that is capable of rotating around the plunger and that is secured on the inside at the front end of the drum. The length of the ring-shaped member in the pricking direction changes along the circumferential direction thereof, and preferably changes continuously or stepwise. The rear end of plunger has a hitting member that strikes the rear end surface in a portion along the circumferential direction of the ring-shaped member. When the plunger moves forward for pricking, the hitting member on the rear end of plunger strikes a portion of the rear end surface of the ring-shaped member in the drum, and is therefore unable to move further forward. The object which the hitting member provided on the rear end of the plunger strikes can be changed from said portion of the rear end surface of the ring-shaped member to the other portion by causing the ring-shaped member to rotate around the plunger by means of the drum rotation. As a result, it is made possible to change the position of the rear end of the plunger with respect to the pricking direction upon hitting the portion of the rear end surface of the ring-shaped member by the rear end of the plunger.

When the position of the rear end of the plunger relative to the pricking direction changes as described above, a length of a rear portion of the plunger which portion extends behind the rear partition changes. Since the length of the plunger itself is fixed, the change in the position of the rear end of plunger means a change in the length of a front portion of the plunger which portion extends ahead from the rear partition, hence a change in the distance between the rear partition and the distal end portion of the pricking member that protrudes from the lancet body grasped by the plunger. In the meantime, since the distance between the rear partition and the front end opening of the case body is fixed, the length of the distal end portion of the pricking member protruding beyond the front end opening of the case body, namely the depth of pricking changes.

It is preferable to provide a cap-like knob that can be fitted in the circumference of the drum, so that the drum can be turned easily from the outside of the injector. The portion of the rear end surface of the ring-shaped member which is hit by the hitting member can be selected by turning the knob.

With reference to the drawing that depicts the injector described above, for example Fig. 34, one embodiment of the injector of the present invention has the pricking depth adjusting drum 360 disposed behind the rear partition 358 of the injector housing adjacent thereto. A pricking depth adjusting ring member 361 having an opening 363 is disposed inside of the drum 360 (these members are preferably integrally connected, and in the embodiment illustrated, for example, the ring is fitted in the drum). The rear end portion 362 of the plunger extends through the opening 363, and the hitting member 365 is provided on the rear end of the plunger. The ring-shaped member 361 is formed so as to be capable of rotating around the plunger 310. Specifically, the drum 360 and the ring-shaped member 361 are caused to rotate around the plunger by turning the cap-like knob that serves as the turning knob 366 fitted onto a rear portion of the drum 360.

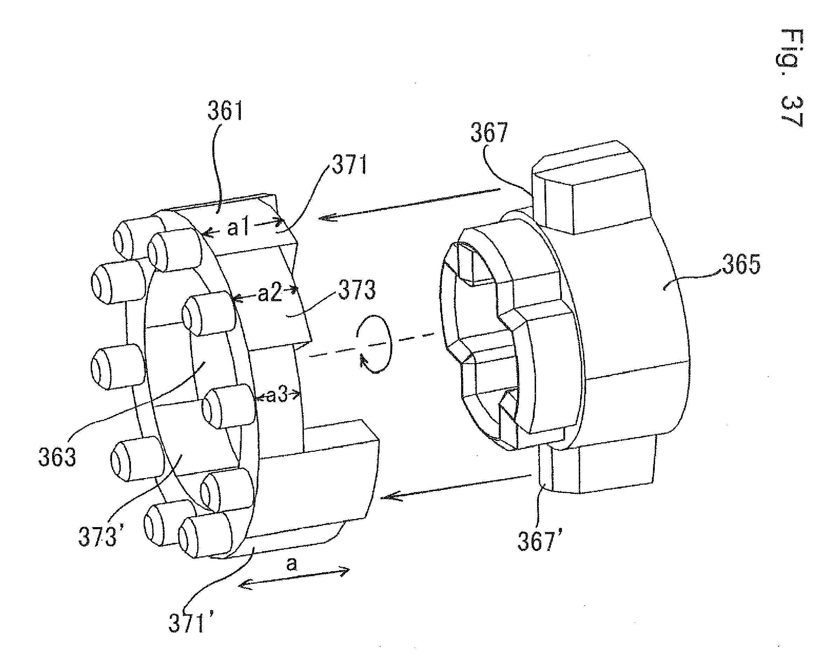

Fig. 37 is a schematic perspective view of the ring-shaped member 361 located around the plunger and a hitting member 365 located at the rear end of the plunger with omitting the plunger (only its extending direction is shown). The length (a) of the ring-shaped member 361 in the pricking direction changes along the circumferential direction thereof continuously or stepwise. In the embodiment illustrated, the length in the pricking direction changes stepwise in six steps as al, a2, and a3. The hitting member 365 of the plunger has hitting members 367 and 367' on both of the upper and the lower sides thereof. When the lancet is launched and the plunger moves forward, the rear end portion 365 moves in the pricking direction as indicated by the arrow, and strikes the ring-shaped member 361.

In the embodiment shown in Fig. 37, for example, when the plunger moves forward, the hitting member 367 strikes the step 371 having a length al in the pricking direction, and the hitting member 367' strikes the step 371' having a length al in the pricking direction. That is, the hitting portion of the hitting member strikes one of the steps that constitute portions of the rear end surface of the ring-shaped member. When the knob 366 is turned a little as indicated clockwise (when viewing from the left side of the drawing) by the arrow in the drawing in this state, the position of the step shifts and the upper hitting portion 367 shifts to the nearer side while the lower hitting position 367 shifts to a farther side.

As a result, the hitting member 367 is allowed to strike the end surface 395 having a length a2 in the pricking direction, and the hitting member 367' is allowed to strike the end surface 398 having a length a2 in the pricking direction.
As will be understood easily, as the lengths in the pricking direction of the ring-shaped member which the hitting portion strikes are different, for example the length in the pricking direction is different between step 371 and step 373, the distance over which the plunger can travel forward, namely the pricking depth changes. In this way, the pricking depth can be controlled by causing the hitting portion to strike other portion of rear end surface of the ring-shaped member which other portion has a different length in the pricking direction.

In the case at least one of the hitting member 365 and the ring-shaped member 361 is formed from an elastic material such as a silicone, an urethane or the like so that it has an impact relieving property, the impact generated by these members when hitting can be mitigated. This provides an advantage of mitigating the impact which the user feels upon the pricking operation.

As will be easily understood from the state of the injector after pricking shown for example in Fig. 26, in which the drum 360 and the ring-shaped member 361 are integrally connected together, pulling the drum rearward causes the ring-shaped member to move rearward and abut against the hitting portion 367, and pulling the knob further rearward thereafter causes the plunger 310 to move rearward so that the protruding in the case where of the plunger moves rearward thus becoming ready to launch.

As a result, after carrying out the pricking operation, or in the case the pricking operation was a failure for some reason (for example, when the lancet was launched before applying a finger tip to the front end of the lancet case), the launchable state can be resumed by pulling back the drum 360 (hence the knob 366), if the lancet assembly is loaded, namely when it has returned to the state as shown in Fig. 26. Then an proper pricking operation can be tried again.

Thus it is preferable that the injector of the present invention is designed such that the launchable state can be achieved by pulling back the drum relative to the injector housing so as to move the plunger rearward. Specifically, with the injector that is in the launchable state, it is made possible for the protrusion of the plunger to pass over the rear end 330 of the trigger lever by pulling back the drum so as to move the plunger rearward. At the instant when the protrusion has passed over the rear end, the trigger lever moves inward, and the lancet becomes able to be launched again.

When the drum is pulled rearward in order to retract the drum as described above, a force that turns the drum may also be exerted, which may accidentally turn the drum around the pricking direction and cause inadvertently setting the pricking depth to an undesirable value. In order to prevent such an unintentional change of the pricking depth, it is preferable that the drum has a rotation limiter located ahead of the rear partition. The rotation limiter comprises a first cylindrical portion that is located ahead of the drum while passing through the rear partition and a second cylindrical portion located ahead of the first cylindrical portion. The plunger passes through the drum rotation limiter and extends in the drum. The drum can move along the longitudinal direction of the plunger together with the drum rotation limiter. It is preferable that the first cylindrical portion can rotate in the opening of the rear partition, but the second cylindrical portion cannot rotate in such opening.

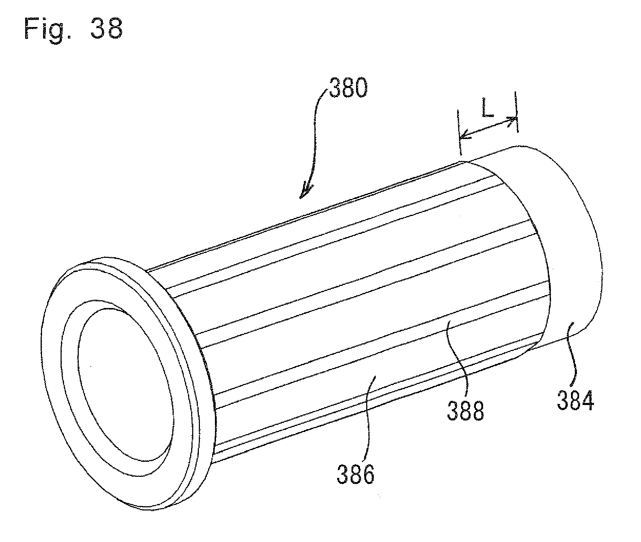

Such rotation limiter can be formed by fitting the rotation limiting member 380 in the connection 382 that extends forward from the drum 360 that is composed of the drum forming components 364 shown in a schematic perspective view of Fig. 38 (only one of a pair of halves as the components is shown). The rotation limiting member 380 comprises a first cylindrical portion 384 that is capable of rotating in the opening defined by the rear partition and a second cylindrical portion 386 located ahead thereof. In one embodiment, the first cylindrical portion 384 and second cylindrical portion are integrated as shown. The plunger extends through the insides of these cylindrical portions.

The second cylindrical portion has, for example, a profile of an equilateral polygonal shape and the opening has an equilateral polygonal shape that circumscribes the former equilateral polygonal shape or surrounds the equilateral polygonal shape with some gap between them, and the first cylindrical portion has a cross section of a shape that can turn within the opening of the equilateral polygonal shape (for example, circular, oval or equilateral polygonal shape).

In the embodiment illustrated, the second cylindrical portion 386 having a profile of equilateral decagon with the corners chamfered, the chamfered portion denoted as 388. The opening of the rear partition has an equilateral decagonal shape that circumscribes or surrounds, with keeping some distance, the former equilateral decagonal shape, and the first cylindrical portion 384 has a circular cross section that can turn within the opening of the equilateral decagonal shape.

Providing the rotation limiter makes it possible to prevent the inadvertent rotation of the drum due to the second cylindrical portion being disposed in the opening of the rear partition when the drum is moved or pulled rearward relative to the injector for the purpose of charging the injector again. The length L of the first cylindrical portion in the pricking direction is at least substantially the same as the thickness of the rear partition and preferably the same as or a little larger than the thickness of the rear partition. There is not restriction on the length of the second cylindrical portion.

When pulling the drum rearward, it is preferable to compress a spring, and the expansion of the compressed spring is utilized when the pulling force is removed, thereby causing the drum to automatically return to its original positions. Specifically, the spring may be disposed around the drum rotation limiter at a position ahead of the rear partition. More specifically, for example, the return spring S3 may be disposed between the rear partition and the flange 390 provided at the front end of the second cylindrical portion.

It is noted that the present invention provides the lancet and the lancet case that constitute the lancet assembly of the present invention described above, the kit of the lancet and the lancet case for constituting the lancet assembly of the present invention described above, the pricking device composed of the lancet assembly of the present invention described above and the injector of the present invention described above, and the kit of the pricking device constituted from the lancet assembly of the present invention described above and the injector of the present invention described above.

### Industrial Applicability

The lancet, the lancet case and the lancet assembly constituted from these members and the injector of the present invention provide a safer and convenient device.

## Claims

1. A lancet assembly comprising a lancet and a lancet case that houses a portion of the former, wherein
the lancet comprises a lancet body, a lancet cap and a pricking member made of a metal;
the lancet case comprises a case body having a front end opening and a rear end opening, and a pair of wings,
as for the lancet, the pricking member is disposed in the lancet body and the lancet cap while straddling over these members, and the distal end portion of the pricking member is enclosed by the lancet cap;
the lancet body has a protruding portion and a stopper located behind the protruding portion;
each wing comprises a front end portion and a rear end portion and an abutting portion located between them, the front end portion is connected to the case body and the rear end portion is able to be fitted into a side of the case body;
when the rear end portions of the wings are not fitted into the sides of the case body, the rear end portions are located outwardly from the case body, so that the wings protrude backward and obliquely from the sides of the case body; and
when the rear end portions of the wings are fitted into the sides of the case body, the wings extend along the sides of the case body.

2. The lancet assembly according to claim 1 **characterized in that** the lancet cap and the lancet body are integrally connected to each other via a weakened portion, and a rear portion of the lancet cap that is located in front of the weakened portion, the weakened portion and a front portion of the lancet body that is located behind the weakened portion are housed in the lancet case.

3. The lancet assembly according to claim 1 **characterized in that** the lancet cap and the lancet body are formed as independent members, and the rear portion of the lancet cap and the front portion of the lancet body are disposed in the lancet case.

4. The lancet assembly according to any one of claims 1 to 3 **characterized in that** the protruding portion of the lancet body composed of two kinds of the protruding portions which are separated from each other with respect to their front-back direction relationship.

5. The lancet assembly according to any one of claims 1 to 4 **characterized in that** the lancet cap includes a protruding portion 216 which protrudes outward, and a fitting portion 217 which is able to be fit in the front end opening of the lancet cap is located behind the protruding portion.

6. The lancet assembly according to claim 4 **characterized in that** a rear portion of the fitting portion is comprises a portion in the form of narrowing rearward.

7. The lancet assembly according to any one of claims 1 to 6 **characterized in that** an abutting portion of the wing is in the form of a step or an portion.

8. The lancet assembly according to any one of claims 1 to 7 **characterized in that** the stopper of the lancet body is in the form of a claw or a fold-back which extends forward and obliquely from the lancet body.

9. The lancet assembly according to any one of claims 1 to 8 **characterized in that** the side of the lancet case comprises a recess which receives the wing, and a rear end portion of the wing is fitted into a space provided on the side of the lancet case by means of press fitting or snap fitting.

10. The lancet assembly according to any one of claims 1 to 9 **characterized in that** a shape of the rear end portion of the wing is a triangle wing shape which gradually flares outward toward its rear.

11. The lancet assembly according to any one of claims 1 to 10 **characterized in that** a thickness of a wall portion which forms the case body and which is located in front of the wing is smaller than that of the other portion of wall portions which form the case body.

12. The lancet assembly according to any one of claims 1 to 11 **characterized in that** one end surface of an end surface of an front end portion of the lancet body and an end surface of a rear end portion of the lancet cap has portions, tops of which form a hypothetical flat surface which extends perpendicular to an extending direction of the pricking element, and the other end surface forms a flat surface which extends perpendicular to the extending direction of the pricking element.

13. The lancet assembly according to any one of claims 1 to 12 **characterized in that** the protruding portion of the lancet body abuts against a wall which defines the rear end opening of the case body when the lancet body is inserted in the lancet case through its front end opening and moved rearward in the lancet case.

14. The lancet assembly according to any one of claims 1 to 13 **characterized in that** the lancet case comprises a portion which gets over a portion provided on an inside of a front end opening of an injector or a portion which fits in a recess provided on the inside of the front end opening of the injector.

15. The lancet assembly according to any one of claims 1 to 14 **characterized in that** the lancet case is formed by injection molding of a resin, so that the front end portion of the stopper is connected integrally to the case body.

16. The lancet assembly according to any one of claims 1 to 15 **characterized in that** the lancet body comprises a rear end portion which fits in a front end portion of a plunger which launches the lancet, the rear end potion comprises a portion or a recess on the outside thereof, and the portion or the recess fits complementally in or with a recess or a portion provided in an inside of the front end portion of the plunger.

17. An injector onto which the lancet assembly according to any one of claims 1 to 16 is loaded so as to launch the lancet body with a distal end portion of the pricking member being exposed, **characterized in that**
the injector comprises therein a plunger which launches the lancet body with the pricking member of which distal end portion is exposed as well as a wing splaying member which expands each wing outward, and
when the lancet assembly of which wings are fitted into the sides of the case body is inserted into the injector through its front end opening, and the rear end portion of the lancet body is moved backward so as to make it abut against the front end portion of the plunger followed by further moving the lancet body backward, the rear end portion of the lancet is fitted into and grasped by the front end portion of the plunger, then
when the lancet body is further moved backward, the trigger lever is in a condition for being able to hold the plunger, then
when the lancet body is further moved backward, the wings is in the state wherein the they have been splayed outward by means of the wing splaying member, then
when a force which moves the lancet body backward is released, a launchable state is achieved, so that loading of the lancet assembly is completed.

18. An injector onto which the lancet assembly according to any one of claims 1 to 16 is loaded so as to launch the lancet body with a distal end portion of the pricking member being exposed, **characterized in that**
the injector comprises therein a plunger which launches the lancet body with the pricking member of which distal end portion is exposed as well as a wing splaying member which expands each wing outward, and
when the lancet assembly of which wings are fitted into the sides of the case body is inserted into the injector through its front end opening, and the rear end portion of the lancet body is moved backward so as to make it abut against the front end portion of the plunger followed by further moving the lancet body backward, the rear end portion of the lancet is fitted into and grasped by the front end portion of the plunger, then
when the lancet body is further moved backward, the trigger lever is in a condition for being able to hold the plunger, and also the wings is in the state wherein the they have been splayed outward by means of the wing splaying member,
in such state just mentioned, when a force which moves the lancet body backward is released, a launchable state is achieved, so that loading of the lancet assembly is completed.

19. The injector according to claim 17 or 18 **characterized in that** the wing splaying member comprises a pair of members which form slopes.

20. The injector according to claim 19 **characterized in that** the pair of the members which form the slopes are wedge members having a tapered off form which are separated from each other.

21. The injector according to any one of claims 17 to 20 **characterized in that** the injector further comprises an ejector which is able to apply a forward force to the lancet case of the loaded lancet assembly.

22. The injector according to claim 21 **characterized in that** the ejector comprises an acting portion, which applies a forward force to the rear end portion of the lancet case, the acting portion abuts against a rear end portion of the lancet case of the lancet assembly which has been loaded.

23. The injector according to claim 22 **characterized in that** the acting portion of the ejector abuts against a rear end portion of a side surface of the lancet case which side surface is adjacent to a side surface on which the wing is located.

24. The injector according to any one of claims 17 to 23 **characterized in that** the plunger comprises a launching spring around itself between a portion provided around its intermediate portion and a rear partition provided in an injector housing,
the launching spring is compressed by the plunger which is moved rearward after the rear end portion of the lancet body is grasped by the plunger, and
when the portion of the plunger is moved rearward so get over a rear end of the trigger lever which is configured to apply a force toward the plunger, the rear end of the trigger is moved to the plunger, so that the trigger lever is in a condition for being able to hold the plunger.

25. The injector according to claim 24 **characterized in that** in the launchable state, the protruding portion of the lancet cap which protruding outward is located separately from the front end opening of the case body, so that substantially whole of the fitting portion in the rear portion of the lancet cap located is located outside the front end opening of the case body.

26. The injector according to claim 24 **characterized in that** in the launchable state, the protruding portion of the lancet cap which protruding outward is located separately from the front end opening of the case body, so that a portion of a narrowing form is located behind the front end opening of the case body.

27. The injector according to any one of claims 17 to 26 **characterized in that** a lower edge of the trigger lever comprises a protrusion or a recess in front of its rear end, and a protruding portion of the plunger is configured such that it is engaged with the protrusion of the trigger lever or it fits in the recess before being engaged with the rear end of the trigger lever for launching the lancet and after launching the lancet.

28. The injector according to any one of claims 17 to 26 **characterized in that** a lower edge of the trigger lever comprises a protruding portion in front of its rear end and a recess which is located behind and adjacent to the protrusion, and a protruding portion of the plunger is configured such that it is engaged with the protrusion of the trigger lever or it fits in the recess before being engaged with the rear end of the trigger lever for launching the lancet and after launching the lancet.

29. The injector according to any one of claims 17 to 28 **characterized in that**
the injector comprises a pricking depth adjusting drum disposed adjacent to a rear partition in an injector housing,
the plunger extends and terminates within the drum, and the drum is capable of rotating around the plunger,
the drum comprises a ring-shaped member which is secured on the inside of the drum and which is rotatable around the plunger, and the length of the ring-shaped member in the pricking direction changes along its circumferential direction,
the rear end of the plunger has a hitting member that strikes the rear end surface of ring-shaped member in its portion along a circumferential direction of the ring-shaped member,
when the plunger moves forward for pricking, the hitting member on the rear end of plunger strikes a portion of the rear end surface of the ring-shaped member in the drum, and
said portion of the rear end surface of the ring-shaped member as an object which the hitting member strikes is able to be changed to the other portion by causing the ring-shaped member to rotate around the plunger by means of the drum rotation.

30. The injector according to claim 29 **characterized in that** the ring-shaped member comprises an impact buffering property.

31. The injector according to claim 29 or 30 **characterized in that** the drum further comprises a drum rotation limiter which comprises a first cylindrical portion extending ahead of the rear partition while passing through the partition and a second cylindrical portion located ahead of the first cylindrical portion, and
the plunger extends in the pricking depth adjusting drum while passing through the drum rotation limiter, and the drum moves along a longitudinal direction of the plunger together with the drum rotation limiter, the first cylindrical portion is able to rotates in an opening of the rear partition, but the second cylindrical portion is not able to rotate in the opening.

32. The injector according to claim 31 **characterized in that** the first cylindrical portion and the second cylindrical portion are integrated to be a single member.

33. A lancet which forms the lancet assembly according to any one of claims 1 to 16.

34. A lancet case which forms the lancet assembly according to any one of claims 1 to 16.

35. A kit of a lancet and a lancet case which are for the formation of the lancet assembly according to any one of claims 1 to 16.

36. A pricking device which is formed of the lancet assembly according to any one of claims 1 to 16 and the injector according to any one of claims 17 to 32.

37. A kit of a pricking device comprising the lancet assembly according to any one of claims 1 to 16 and the injector according to any one of claims 17 to 32.
